(19) Europäisches Patentamt
European Patent Office
Office européen des brevets

(11) Veröffentlichungsnummer: 0 298 385 A2

# (12) EUROPÄISCHE PATENTANMELDUNG

(21) Anmeldenummer: 88110522.5

(51) Int. Cl.4: C07H 15/252 , A61K 31/70

(22) Anmeldetag: 01.07.88

Patentansprüche für folgende Vertragsstaaten: ES + GR.

(30) Priorität: 09.07.87 DE 3722699

(43) Veröffentlichungstag der Anmeldung: 11.01.89 Patentblatt 89/02

(84) Benannte Vertragsstaaten: AT BE CH DE ES FR GB GR IT LI LU NL SE

(71) Anmelder: BEHRINGWERKE Aktiengesellschaft
Postfach 1140
D-3550 Marburg 1(DE)

(72) Erfinder: Hermentin, Peter, Dr.
Barfüssertor 30
D-3550 Marburg(DE)
Erfinder: Gronski, Peter, Dr.
Kirchgraben 5
D-3551 Lahntal(DE)
Erfinder: Hoffmann, Dieter, Dr.
Im Stetefeld 6
D-3551 Lahntal(DE)
Erfinder: Lüben, Gerhard, Dr.
An der Haustatt 25
D-3550 Marburg(DE)
Erfinder: Kraemer, Hans Peter, Dr.
Birkenweg 16
D-3550 Marburg(DE)
Erfinder: Bosslet, Klaus, Dr.
Am Schlag 5
D-3550 Marburg(DE)

(74) Vertreter: Meyer-Dulheuer, Karl-Hermann, Dr. et al
HOECHST Aktiengesellschaft Zentrale
Patentabteilung Postfach 80 03 20
D-6230 Frankfurt/Main 80(DE)

## (54) Zytostatisch wirksame Anthracyclin-Derivate.

(57) Die Erfindung betrifft neue zytostatisch wirksame Anthracyclinderivate und -konjugate der nachfolgenden allgemeinen Formel I,

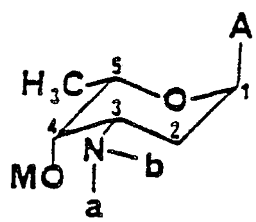

I

welche gegebenenfalls als Salz einer anorganischen oder organischen Säure vorliegen, in der die Reste

EP 0 298 385 A2

folgende Bedeutung haben: A ist ein Anthracyclinon der allgemeinen Formel II oder III,

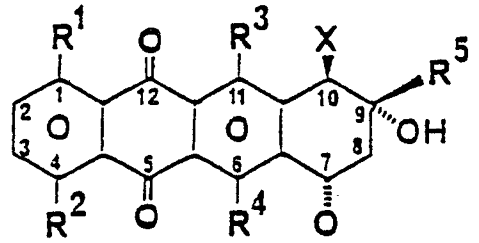

II

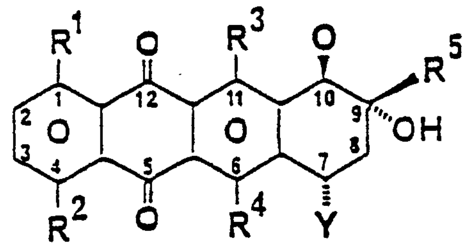

III

M ist eine Struktur der allgemeinen Formel IV oder V,

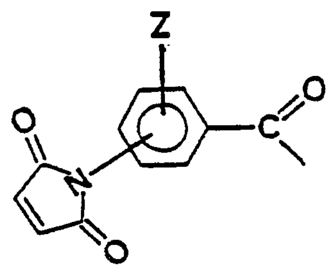

IV

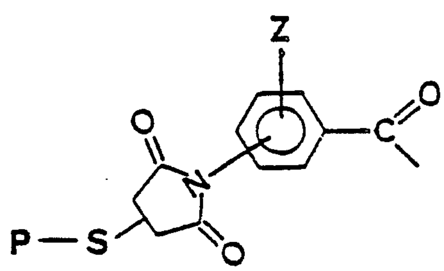

V

und a und b sind gleichartige oder verschiedenartige substituierte oder unsubstituierte Alkylreste aliphatischer oder benzylaromatischer Natur oder a und b bilden, zusammen mit dem N-Atom, einen gegebenenfalls substituierten heterozyklischen Ring,
wobei in den Formeln II und III
$R^1$ Wasserstoff oder eine Hydroxygruppe,
$R^2$ Wasserstoff oder eine Hydroxy- oder eine Methoxygruppe,
$R^3$ Wasserstoff oder eine Hydroxygruppe,
$R^4$ Wasserstoff oder eine Hydroxygruppe,
$R^5$ $CH_2CH_3$, $COCH_3$ oder eine Strukrur der Formel $COCH_2OCOR$, wobei R zusammen mit der Carbonylgruppe die Acylfunktion einer einbasigen oder einer α,ω-zweibasigen aliphatischen Carbonsäure oder einer Aminosäure darstellt,

X Wasserstoff, eine Hydroxy- oder eine Methoxycarbonylgruppe oder ein Rest der Formel VI

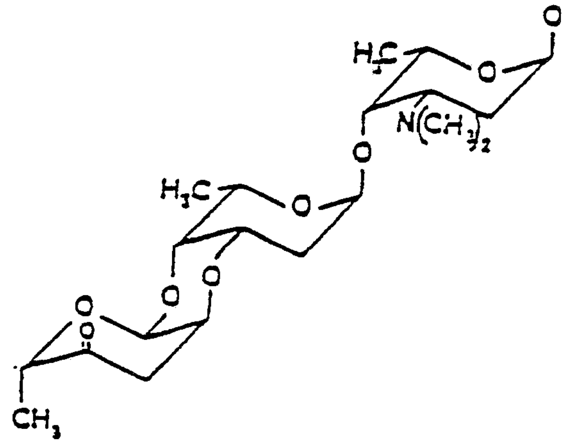

VI

und Y ein Rest der Formel VI ist,
wobei in den Formeln IV und V der Rest Z Wasserstoff oder eine Methyl-, Hydroxy-, Methoxy-, Acetoxy-, Nitro- oder Cyanogruppe oder ein Chlor- oder Bromatom sein kann und wobei in Formel V der Rest P ein Protein- oder Peptid-Rest, ein Immunglobulin oder Immunglobulin-Rest, ein monoklonaler Antikörper oder ein Fab-, Fab'- oder F(ab')$_2$-Fragment oder ein Hormon-, Lectin- oder Wachstumsfaktorrest sein kann, wobei P gegebenenfalls weitere Anthracyclinderivate der beanspruchten gekoppelten Art tragen kann, sowie Verfahren zu ihrer Herstellung und ihre Verwendung in Arzneimitteln.

## Zytostatisch wirksame Anthracyclin-Derivate

Die Erfindung betrifft neue, zytostatisch wirksame Anthracyclinderivate einschließlich Anthracyclin-Konjugate, Verfahren zu ihrer Herstellung sowie ihre Verwendung in Arzneimitteln.

Die Substanzklasse der Anthracycline ist schon lange bekannt und seit der Strukturermittlung der Rhodomycine, des Adriamycins bzw. des Daunomycins und dem Bekanntwerden der zytostatischen Wirksamkeit gewisser Vertreter dieser Anthracyclinklasse wurde eine Vielzahl von Anthracyclinen auf biologischem Wege aus Vertretern der Actinomyceten-Gattung Streptomyces isoliert und in ihrer Wirksamkeit erforscht.

Es ist bekannt, daß die Anthracycline Daunomycin und Adriamycin und Anthracycline, welche von diesen beiden Grundstrukturen abgeleitet sind, wie z.B. 4′-epi-Adriamycin, 4′-Methoxyadriamycin oder 4′-Desoxyadriamycin bereits für die Therapie von Tumoren verwendet werden.

Es ist jedoch auch bekannt, daß die genannten Anthracycline nur bei schnell wachsenden Tumoren, z.B. bei Leukämie, gute Wirksamkeit entfalten, nicht jedoch bei den langsam wachsenden soliden Tumoren, z.B. den Carcinomen des Colon oder des Pankreas.

Bei der tumortherapeutischen Verwendung dieser bekannten Anthracycline liegt ein wesentliches Problem darin, daß sie neben der gewünschten zytostatischen Wirksamkeit unerwünschte Nebenwirkungen aufweisen, wie beispielsweise eine hämatologische oder cardiale Toxizität, und daß Tumore nach einer gewissen Zeit gegen das zur Therapie verwendete Anthracyclin, zumeist Adriamycin, resistent werden.

Ausgehend von diesem Stand der Technik ist es Aufgabe der vorliegenden Erfindung, neue, nicht kreuzresistente Anthracyclinderivate zu schaffen, die sich durch ein neues Wirkungsspektrum und eine geringere Toxizität auszeichnen. Weiterhin ist es Aufgabe der vorliegenden Erfindung, die neu geschaffenen Anthracycline in geeigneter Weise an monoklonale Antikörper und dergleichen anzuknüpfen, welche gegen Tumore gerichtet sind, und hierfür Spacer zu entwickeln, welche unter geeigneten Bedingungen eine Freisetzung des an den Antikörper gebundenen Zytostatikums erlauben.

Gelöst wird diese erfindungsgemäße Aufgabe mit neuen zytostatisch wirksamen Anthracyclinderivaten und -konjugaten der nachfolgenden allgemeinen Formel I,

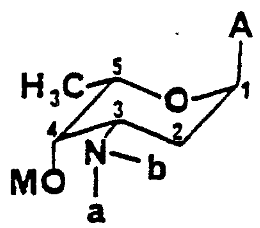

I

welche gegebenenfalls als Salz einer anorganischen oder organischen Säure vorliegen, in der die Reste folgende Bedeutung haben:

A ist ein Anthracyclinon der allgemeinen Formel II oder III,

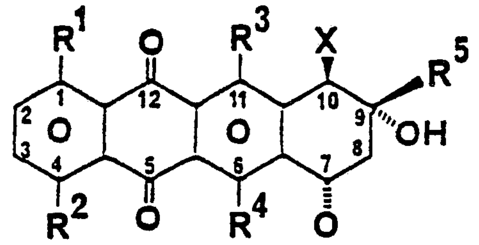

II

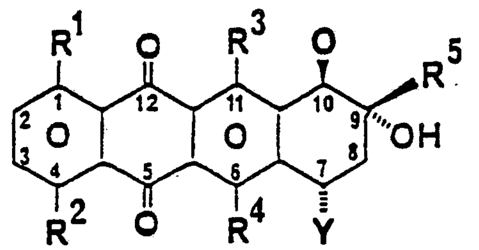

III

M ist eine Struktur der allgemeinen Formel IV oder V,

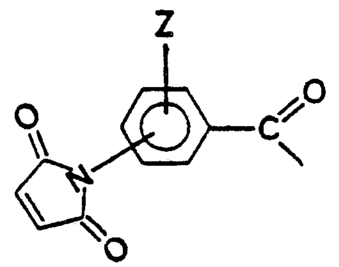

IV

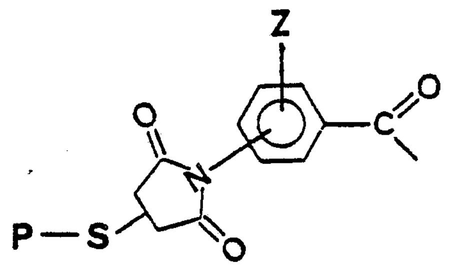

V

und a und b sind gleichartige oder verschiedenartige substituierte oder unsubstituierte Alkylreste aliphatischer oder benzylaromatischer Natur, und sie können insbesondere $C_1$-$C_8$-Alkyl, Cyanomethyl, Benzyl oder in ortho-, meta-oder para-Stellung durch Methyl, Ethyl, Hydroxy, Methoxy, Ethoxy, Nitro, Cyano, Fluor, Chlor oder Brom substituiertes Benzyl sein
oder a und b bilden, zusammen mit dem N-Atom einen gegebenenfalls substituierten heterozyklischen Ring, bevorzugt eine Morpholino- oder Cyanomorpholino-Struktur,
wobei in den Formeln II und III
$R^1$ Wasserstoff oder eine Hydroxygruppe,
$R^2$ Wasserstoff oder eine Hydroxy- oder eine Merhoxygruppe,

$R^3$ Wasserstoff oder eine Hydroxygruppe,
$R^4$ Wasserstoff oder eine Hydroxygruppe,
$R^5$ $CH_2CH_3$, $COCH_3$, oder eine Struktur der Formel $COCH_2OCOR$, wobei R zusamm en mit der Carbonylgruppe die Acylfunktion einer einbasigen oder einer α, ω-zweibasigen aliphatischen Carbonsäure oder einer Aminosäure darstellt,
X Wasserstoff, eine Hydroxy- oder eine Methoxycarbonylgruppe oder ein Rest der Formel VI

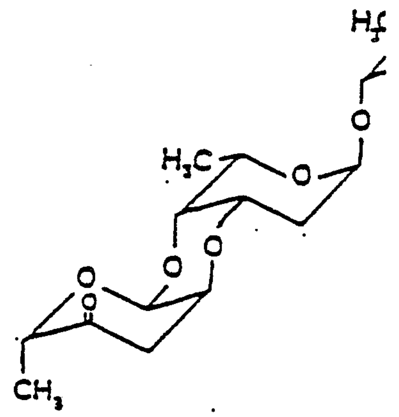

und Y ein Rest der Formel VI ist,
wobei in den Formeln IV und V der Rest Z Wasserstoff oder eine Methyl-, Hydroxy-, Methoxy-, Acetoxy-, Nitro- oder Cyanogruppe oder ein Chlor- oder Bromatom sein kann und wobei in Formel V der Rest P ein Protein- oder Peptid-Rest, insbesondere ein Immunglobulin oder Immunglobulin-Rest, bevorzugt ein monoklonaler Antikörper oder ein Fab -, Fab′ oder ein F(ab′)$_2$ Fragment sein kann, wobei P gegebenenfalls weitere Anthracyclinderivate der beanspruchten gekoppelten Art tragen kann. P kann insbesondere auch ein Hormon-, Lectin- oder Wachstumsfaktorrest sein. Als Anthracyclin-Konjugate werden die Anthracyclin-Derivate der Formel I bezeichnet, bei denen M die Struktur V hat.

Eines der Konzepte zur Therapie von Tumoren sieht vor, eine zytostatische Substanz, welche in der Lage ist, das Wachstum von Tumoren zu verlangsamen oder zu unterbinden, mittels eines geeigneten Carriers, beispielsweise mittels eines monoklonalen Antikörpers, welcher gegen den jeweiligen Tumor gerichtet ist, direkt an den erwünschten Wirkort, nämlich den jeweiligen Tumor, heranzuschleppen. Dort kann die an den Carrier gekoppelte Substanz unter geeigneten Bedingungen ihre zytostatische Wirkung gegen den Tumor sehr gezielt entfalten. Gleichzeitig wird durch die Kopplung der zytostatischen Substanz an den Antikörper die allgemein-toxische Wirkung der Substanz auf das Wirtsgewebe vermindert. Solche zytostatischen Substanzen können beispielsweise Vinca-Alkaloide, Anti-Metabolite, Anthracycline oder pflanzliche oder bakterielle Toxine sein (siehe Gallego & Price, Drugs of Today (1985), 21, 511).

Es ist ferner bekannt, daß eine zytostatische Substanz, welche an einen Carrier gekoppelt ist, zur Entfaltung ihrer zytostatischen Wirkung in der Zelle wieder aus dem Konjugat abgespalten werden muß (Gallego & Price, Drugs of Today (1985), 21, 511).

Es ist auch bekannt, daß die Anthracycline Daunomycin und Adriamycin, wenn sie in geeigneter Weise an ein Protein gekoppelt sind, eine geringere allgemeine Toxizität aufweisen als in gelöster Form und daß sie, wenn sie in geeigneter Weise an ein Immunglobulin gekoppelt sind, in bestimmten Fällen und im Vergleich zu einer geeigneten Kontrolle das Wachstum von Tumoren verlangsamen können (siehe z. B. Gallego & Price, Drugs of Today (1985), 21, 511).

Für die Anknüpfung therapeutischer Agenzien sind zahlreiche Kopplungsvarianten bekannt (siehe z. B. Gallego & Price, Drugs of Today (1985), 21, 511).

In WO 86/01409 vom 23.05.85 (Blattler et al.) werden Maleimido-Spacer beschrieben, welche eine säurelabile Amidbindung enthalten, was - bei genügend saurem pH -eine Freisetzung des therapeutischen Agens auf hydrolytischem Wege erlaubt.

Es ist andererseits auch bekannt, daß eine m-Maleimidobenzamid-Spacerbindung nicht spaltbar ist (Gallego & Price, Drugs of Today (1985), 21, 511).

Ausgehend von diesem vorstehend abgehandelten Stande der Technik wurde nun überraschenderweise gefunden, daß sich Verbindungen der Formel I mit M = H selektiv an der 4′-Hydroxygruppe des Zuckersegments mit einer Maleimido-Funktion verestern lassen, ohne daß die anderen in der Verbindung der Formel I vorhandenen alkoholischen oder phenolischen Hydroxygruppen mitacyliert werden.

Zu dieser selektiven Veresterung an der 4′-Hydroxygruppe des Zuckersegments eignen sich insbesondere Maleimido-Derivate von Carbonsäuren.

Weiterhin wurde überraschenderweise gefunden, daß die in Position 4′ des Zuckerbausteins eingeführte Ester-bindung - je nach Beschaffenheit des Spacers - wieder hydrolysiert werden kann, ohne daß hierzu hydrolytische Enzyme, wie z.B. Esterasen, oder saure Bedingungen erforderlich sind. Dies stellt einen entscheidenden Vorteil der Erfindung dar.

So wurde beispielsweise gefunden, daß sich Cytorhodin-S, welches mittels eines 4-Maleimidobenzoyl-Spacers über die freie OH-Gruppe des Rhodosamin-Zuckerbausteins an einen monoklonalen Antikörper angeknüpft wurde, in wäßriger Lösung wieder aus dem Konjugat freisetzen läßt.

Überraschenderweise wurde auch gefunden, daß die Maleimido-Esterbindung zu Rhodosamin bedeutend stabiler ist, wenn anstelle einer aliphatischen Maleimido-Carbonsäure eine aromatische Maleimido-Carbonsäure, wie z.B. Maleimido-Benzoesäure, als Spacer verwendet wird (Fig. 1).

Weiterhin wurde gefunden, daß die Hydrolysierbarkeit des Maleimido-Benzoesäureesters in Position 4′ des Zuckerbausteins Rhodosamin durch geeignete Substituenten im Phenylenring des Spacers variiert werden kann, wodurch sich die Geschwindigkeit der Freisetzung des Anthracyclins aus dem Konjugat bis zu einem gewissen Grad steuern läßt (siehe Fig. 1).

Ferner wurde gefunden, daß sich die hergestellten Maleimidoacyl-Derivate von Anthracyclinen problemlos an Thiolgruppen von Proteinen bzw. Antikörpern koppeln lassen, wobei die Thiolgruppen im Protein entweder bereits nativ vorhanden oder auf chemischem Wege eingeführt oder durch Reduktion von Disulfidbindungen erzeugt werden können. Es wurde damit eine prinzipiell neue und generalisierbare Methode zur Kopplung von Anthracyclinen der Formel I an die SH-Gruppen von Proteinen bzw. Antikörpern entwickelt. Hierin liegt ein weiterer besonderer Vorteil der vorliegenden Erfindung.

Ferner wurde gefunden, daß sich die durch selektive Reduktion der Interketten-Disulfid-Bindungen von Immunglobulin erzeugten freien SH-Gruppen in hervorragender Weise zur Anknüpfung von Anthracyclinen eignen.

Die Vorteile des vorgestellten neuen Bindungstyps zur Kopplung von Rhodosaminyl-Anthracyclinonen lassen sich wie folgt zusammenfassen:

1. Die Bindung des Anthracyclins an Antikörper über einen Maleimido-Spacer erfolgt in reversibler bzw. hydrolysierbarer Ester-Bindung.

2. Die Bindung des Maleimido-Spacers an Antikörper erfolgt selektiv zu den durch Reduktion der Interketten-Disulfidbindung von Antikörpern erzeugten Thiolgruppen, vermeidet also die Nachteile einer Kopplung an auf chemischem Weg (unter Ausbildung einer Amidbindung) in den Antikörper eingeführten SH-Gruppen.

3. Die Bindungs-Methode ist gültig für Anthracycline der Formel I und eröffnet somit ein generalisierbares Kopplungsprinzip.

4. Das gebundene Anthracyclin wird unter physiologischen Bedingungen bzw. in wäßrigem Medium durch einfache Hydrolyse der Ester-Bindung freigesetzt, erfordert zur Bindungsspaltung also keine Enzyme.

5. Die Geschwindigkeit der Freisetzung des esterartig gebundenen Anthracyclins aus dem Konjugat läßt sich durch geeignete Substituenten im Phenylen-Kern des Spacer-Elements in geeigneter Weise steuern.

6. Freigesetzt wird letzlich jeweils das unveränderte Anthracyclin, der Spacer verbleibt in chemischer Bindung am Antikörper.

Ausgehend von diesen Feststellungen haben sich die in den Unteransprüchen beanspruchten Anthracyclinderivate als besonders vorteilhaft erwiesen. Das erfindungsgemäße Verfahren zur Herstellung der neuen zytostatisch wirksamen Anthracyclinderivate und Anthracyclin-Konjugate ist dadurch gekennzeichnet, daß man

eine Verbindung der Formel I, in welcher A ein Anthracyclinon der Formel II oder III mit $R^1 = H$ oder OH, $R^2 = H$, OH oder $OCH_3$, $R^3 = H$ oder OH, $R^4 = H$ oder OH, $R^5 = CH_2CH_3$, $COCH_3$ oder eine Struktur der Formel $COCH_2OCOR$, wobei R zusammen mit der Carbonylgruppe die Acylfunktion einer einbasigen oder einer $\alpha$, $\omega$-zweibasigen aliphatischen Carbonsäure oder einer Aminosäure darstellt, X = H, OH, $COOCH_3$ oder ein Rest der Formel VI und Y ein Rest der Formel VI ist

und in welcher a und b gleichartige oder verschiedenartige substituierte oder unsubstituierte, Alkylreste aliphatischer oder benzylaromatischer Natur, insbesondere $C_1$-$C_8$-Alkyl, Cyanomethyl, Benzyl oder in ortho-, meta-oder para-Stellung durch Methyl, Ethyl, Hydroxy, Methoxy, Ethoxy, Nitro, Cyano, Fluor, Chlor oder Brom substituiertes Benzyl sind oder in welchen a und b zusammen mit dem N-Atom einen gegebenenfalls substituierten heterozyklischen Ring, vorzugsweise eine Morpholino- oder Cyanomorpholino-Struktur darstellen,

und in welcher M = H ist,

mit einem reaktionsfähigen gegebenenfalls im aromatischen Ring durch $CH_3$, OH, $OCH_3$, $OCOCH_3$, $NO_2$, CN, Cl oder Br substituierten Maleimidobenzoesäurederivat, z.B. dem entsprechenden Säurechlorid oder -bromid, bevorzugt mit einem reaktionsfähigen, gegebenenfalls substituierten ortho-oder para-Maleimidobenzoesäurederivat und besonders bevorzugt mit einem reaktionsfähigen para-Maleimidobenzoesäurederivat in einem Zweiphasensystem, bestehend aus einem mit Wasser nicht mischbaren organischen Lösungsmittel, wie beispielsweise Chloroform oder Dichlormethan, und einer wäßrigen Phase, welche eine geeignete Base, wie beispielsweise Natriumhydrogencarbonat, enthält, vorzugsweise unter Rühren bei einer Temperatur zwischen beispielsweise 0° C und 25° C im Dunkeln zu einem Produkt der Formel I umsetzt, in welchem A, a, b, $R^1$, $R^2$, $R^3$, $R^4$, $R^5$, X und Y die genannte Bedeutung haben und in welchem M eine Struktur der Formel IV ist, in welcher Z die genannte Bedeutung hat, dieses Produkt aus der organischen Phase isoliert und aufarbeitet.

Gegebenenfalls wird das so erhaltene Produkt mit einem SH-Gruppen tragenden Protein- oder Peptid-Rest, insbesondere einem SH-Gruppen tragenden Immunglobulin oder Immunglobulin-Rest, bevorzugt mit einem Immunglobulin oder Immunglobulin-Rest, in welchem die SH-Gruppen durch Reduktion der Interketten-Disulfid-Bindungen erzeugt wurden, bevorzugt mit einem SH-Gruppen tragenden monoklonalen Antikörper, bevorzugt mit einem monoklonalen Antikörper, in welchem die SH-Gruppen durch Reduktion der Interketten-Disulfid-Bindungen erzeugt wurden, oder ebenfalls bevorzugt mit einem Fab- oder Fab'-Fragment eines Antikörpers, bevorzugt eines monoklonalen Antikörpers, oder aber mit künstlich eingeführten SH-Gruppen versehenen Stoffen der vorgenannten Art in einem geeigneten wäßrigen Puffersystem, beispielsweise in einer physiologischen Kochsalzlösung oder einem Kochsalz enthaltenden Phosphat-Puffer, welches gegebenenfalls bis zu 30 Volumenprozent eines mit Wasser mischbaren organischen Lösungsmittels, wie beispielsweise Dimethylformamid oder Dimethylsulfoxid, ent halten kann, bei einer Temperatur zwischen beispielsweise 0° C und 25° C im Dunkeln bei einem geeigneten pH zwischen beispielsweise pH 5 und pH 9 über einen Zeitraum von beispielsweise 5 Minuten bis 2 Stunden umsetzt, wobei eine Verbindung der Formel I erhalten wird, in welcher A, a, b, $R^1$, $R^2$, $R^3$, $R^4$, $R^5$, X und Y die genannte Bedeutung haben und in welcher M eine Struktur der Formel V ist, in welcher Z die genannte Bedeutung hat und P ein Protein- oder Peptid-Rest, insbesondere ein Immunglobulin oder Immunglobulin-Rest, bevorzugt ein monoklonaler Antikörper ein Fab- oder Fab'-Fragment oder $F(ab')_2$-Fragment, ein Hormon-, Lectin - oder Wachstumsfaktor-Rest sein kann, wobei P gegebenenfalls weitere Anthracyclinderivate der beanspruchten gekoppelten Art tragen kann.

Die nach dem erfindungsgemäßen Verfahren erhaltenen neuen Anthracyclinderivate zeichnen sich durch zytostatische Aktivität aus und können daher zusammen mit den üblichen pharmazeutischen Konfekrionierungs- und/oder Verdünnungsmitteln zu Arzneimitteln verarbeitet werden, die in der Krebstherapie Anwendung finden. Die Dosierungs- und Anwendungsweise entspricht dabei im wesentlichen derjenigen für die bekannten Substanzen Adriamycin, Daunomycin, Aclacinomycin, 4'-epi-Adriamycin, 4'-Methoxyadriamycin oder 4'-Desoxyadriamycin.

Die solchermaßen hergestellten Arzneimittel können zusätzlich noch andere Wirkstoffe enthalten, sofern diese zusammen mit den erfindungsgemäßen Verbindungen keine unerwünschten Nebenwirkungen zeigen.

Die nach dem erfindungsgemäßen Verfahren erhaltenen Anthracyclin-Antikörper-Konjugate enthalten in der Regel 6 bis 10 Mol Anthracyclin je Mol Antikörper, ausreichend zur Entfaltung einer geeigneten zytostatischen Wirkung.

Die klinische Dosis zur Applikation der Anthracyclin-Antikörper-Konjugate kann - in Abhängigkeit von dem gekoppelten Anthracyclin, dem zu behandelnden Tumor, dem verwendeten Antikörper und der Administration - variieren und kann beispielsweise bei einem Cytorhodin-S-Konjugat zwischen beispielsweise 1 bis 20 mg Konjugat pro Tag pro Erwachsenem liegen.

Das Konjugat kann beispielsweise intravenös, intraperitoneal oder rectal verabreicht werden.

Pharmazeutische Präparate, welche ein Anthracyclin-Antikörper-Konjugat enthalten, werden nach den in der Immunologie bzw. Medizin üblichen Verfahren hergestellt, wobei die üblichen Träger und Additive verwendet werden können. Die injizierbare Präparation kann z.B. puderförmig sein und neben dem Konjugat einen geeigneten Stabilisator, wie z.B. Mannitol, Saccharose, Lactose, Maltose, Glucose oder Fructose enthalten. Diese Präparationsmischung wird in Wasser gelöst, in Ampullen aufgeteilt, lyophilisiert und verschlossen.

Die rectal applizierbare Präparation kann unter Verwendung einer lipophilen Base, beispielsweise einer Mischung bestehend aus Kakao-Öl oder Fettsäure-Triglyceriden mit einem Fettsäure-Monoglycerid in verschiedenen Mischungsverhältnissen, oder unter Verwendung einer hydrophilen Base, beispielsweise Polyethylenglycol oder Glycerogelatine, hergestellt werden, wobei durch gelindes Erwärmen der Mischung eine Lösung hergestellt wird, welche homogenisiert und appliziert wird.

Die zytostatische Wirksamkeit der erfindungsgemäßen Verbindungen wurde anhand von L1210 Leukämiezellen der Maus getestet. Hierzu wurde die Koloniebildung von L1210 Leukämiezellen in Agarplatten herangezogen. Diese Methode dient zum Nachweis des Einflusses der Testsubstanzen auf das Wachstumsverhalten der Zellen über 1 Stunde oder über mehrere Generationen. Bei einer Zellzykluszeit von 10-12 Stunden werden dabei in der Testzeit von 7 Tagen etwa 14 aufeinanderfolgende Generationen beobachtet. Die erfindungsgemäßen zytostatisch wirksamen Substanzen bewirken in diesem Test eine Reduktion der zu beobachtenden Koloniezahl gegenüber einer unbehandelten Kontrollprobe.

Einzelheiten des Testverfahrens ergeben sich aus der nachfolgenden Verfahrensweise zur Ermittlung der Koloniebildung.

Verfahrensweise zur Ermittlung der Koloniebildung von L1210 Leukämiezellen in Soft-Agar

500 Leukämiezellen pro Platte wurden mit unterschiedlichen Konzentrationen der Testsubstanz 1 Stunde bei 37°C inkubiert. Anschließend wurden die Zellen zweimal mit McCoy5A-Medium gewaschen und schließlich in Petrischalen nach Zugabe von 0,3 % Agar ausgegossen. Kontrollen wurden lediglich mit frischem Medium inkubiert. Anstelle der einstündigen Inkubation wurden in manchen Fällen unterschiedliche Konzentrationen der Testsubstanzen der oberen Agarschicht zugemischt, um so eine kontinuierliche Exposition der Zellen über die gesamte Inkubationszeit zu erreichen. Nach Erstarren des Agars wurden die Platten im Brutschrank 7 Tage bei 37°C inkubiert (5 Vol. -% $CO_2$, 95 % relative Luftfeuchtigkeit). Anschließend wurde die Zahl der entstandenen Kolonien mit einem Durchmesser von mehr als 60 µm gezählt. Die Ergebnisse wurden angegeben als Koloniezahl in behandelten Agarplatten in Prozent der unbehandelten Kontrolle. Aus der so erhaltenen Dosiswirkungskurve wurde die $IC_{50}$ als Maß für die Wirksamkeit der Substanz ermittelt. Die Ergebnisse für die hier beschriebenen Verbindungen im Vergleich zu Adriamycin sind in der nachfolgenden Tabelle 1 zusammengefaßt.

Die Struktur der hergestellten Verbindungen wurde mittels $^1H$-NMR- Spektroskopie, UV-Spektroskopie und HPLC-Technik ermittelt. Der Verlauf der Reaktionen wurde, soweit möglich, dünnschichtchromatographisch auf Kieselgel-Fertigplatten verfolgt. Die Ergebnisse der Zytotoxizitäts-Testung gegen L1210-Zellen in vitro sind in Tabelle 1 zusammengefaßt.

Tabelle 1

| $IC_{50}$-Werte gegenüber L1210-Zellen in vitro | | |
|---|---|---|
| Testsubstanzen | Dauerinkubation[a] (µg/ml) | 1h-Inkubation (µg/ml) |
| Adriamycin | 0,02 | 0,04 |
| β-Rhodomycin-I | 0,022 | 0,05 |
| Cytorhodin-S | 0,003 | 0,013 |
| Verbindung 8 | 0,11 | 0,5 |
| Verbindung 11 | 0,028 | 0,11 |
| Verbindung 12 | 0,0028 | 0,0095 |
| Konjugat aus Beispiel 6 | < 0,04 | 0,19 |
| Konjugat aus Beispiel 7 | < 0,04 | 0,26 |

a) nicht differenziert nach etwaigen Hydrolyseprodukten

Zur Erläuterung des erfindungsgemäßen Herstellungsverfahrens werden nachfolgend Beispiele 1 bis 11 aufgeführt, in denen bevorzugt erfindungsgemäße Verbindungen nach dem beanspruchten Verfahren hergestellt wurden.

Herstellung der Ausgangsmaterialien

4-Maleimidobenzoesäure (Verbindung 1)

5g (36 mmol) 4-Aminobenzoesäure wurden in 30 ml trockenem Aceton suspendiert und durch Zusatz von 5 ml trockenem Methanol in Lösung gebracht. Dann wurde unter kräftigem Rühren eine Lösung von 4,1 g (42 mmol) Maleinsäureanhydrid in 10 ml trockenem Aceton zugetropft und der ausgeflockte Niederschlag abfiltriert und im Vakuum getrocknet (Ausbeute 7,5 g).

1 g dieses Filterkuchens wurde, zusammen mit 170 mg wasserfreiem Natriumacetat, in 2 ml Acetanhydrid 2 h bei 50 °C gerührt. Die entstandene klare Lösung wurde am Rotationsverdampfer eingeengt, mit 30 ml Wasser versetzt und 2 h bei 70 °C gerührt. Die dabei präzipitierende 4-Maleimidobenzoesäure wurde nach Abkühlung auf Raumtemperatur abfiltriert und im Vakuum getrocknet (Ausbeute 556 mg).

$^1$H-NMR (90MHz, $CDCl_3/D_6$-DMSO (3/2), TMS); 8.14 (d, 2H, J = 8.3Hz, H-2, H-6); 7.48 (d, 2H, J = 8.3Hz, H-3, H-5); 6.91 (s, 2H, maleimido-H).

4-Maleimidobenzoylchlorid (Verbindung 2)

700 mg (3,22 mmol) 4-Maleimidobenzoesäure (Verbindung 1) wurden in 30 ml trockenem Toluol suspendiert, mit 4 ml Thionylchlorid versetzt und 5 h bei 100 °C gerührt. Dann wurde am Rotationsverdampfer zur Trockne eingeengt und im Hochvakuum getrocknet (Ausbeute 755 mg).

2-Chlor-4-maleimidobenzoesäure (Verbindung 3)

2 g [11,7 mmol) 4-Amino-2-chlorbenzoesäure wurden in Analogie zu Beispiel 1 mit 1,3 g (13,3 mmol) Maleinsäureanhydrid umgesetzt. Nach erfolgter Flockung wurde noch 1 h bei 4 °C gerührt, das Präzipitat abfiltriert, mit wenig trockenem Aceton nachgewaschen und im Vakuum getrocknet (Ausbeute 3,1 g). Der Filterkuchen wurde, zusammen mit 450 mg wasserfreiem Natriumacetat, in 16 ml Acetanhydrid 1 h bei 60 °C gerührt und die erhaltene klare Lösung analog Beispiel 1 zum gewünschten Produkt aufgearbeitet (Ausbeute 1,13 g).

$^1$H-NMR (90MHz, $CDCl_3/D_6$-DMSO (4/1), TMS): 7,97 (d, 1H, $J_{5,6}$ = 9Hz, H-6); 7,60 (d, 1H, $J_{3,5}$ = 2.4Hz, H-3); 7.46 (dd, 1H, $J_{5,6}$ = 9Hz, $J_{3,5}$ = 2.4Hz, H-5); 7.09 (s, 2H, maleimido-H)

2-Chlor-4-maleimidobenzoylchlorid (Verbindung 4)

1 g (3,97 mmol) 2-Chlor-4-maleimidobenzoesäure (Verbindung 3) wurden in einer Mischung aus 30 ml trockenem Toluol und 8 ml Thionylchlorid 3 h bei 120 °C gerührt. Dann wurde am Rotationsverdampfer zur Trockne eingeengt und im Hochvakuum getrocknet (Ausbeute 1,07 g).

2-Acetoxy-4-maleimidobenzoesäure (Verbindung 5)

4,6g(30 mmol) 4-Amino-2-hydroxybenzoesäure wurden in Analogie zu Beispiel 3 mit 3,4 g (34,7 mmol] Maleinsäureanhydrid umgesetzt und aufgearbeitet. Der erhaltene Filterkuchen (6,26 g) wurde in Analogie zu Beispiel 1 mit 1 g Natriumacetat in 35 ml Acetanhydrid 1 h bei 60 °C gerührt, filtriert, und das Lösungsmittel abgezogen. Der gelartige Rückstand wurde in 200 ml Wasser 2 h bei 70 °C gerührt. Nach Abkühlung auf Raumtemperatur wurde der entstandene Niederschlag über eine Glasfritte ($P_3$) abfiltriert, mehrmals mit Wasser nachgewaschen und getrocknet (Ausbeute 0,91 g).

$^1$H-NMR (90MHz, $CDCl_3/D_6$-DMSO (3/1), TMS): 8.11 (d, 1H, $J_{5,6}$ = 9Hz, H-6); 7.45 (dd, 1H, $J_{5,6}$ = 9Hz, $J_{3,5}$ = 1.5Hz, H-5); 7.27 (d, 1H, $J_{3,5}$ = 1.5Hz, H-3); 7.1 (s, 2H, maleimido-H); 2.31 (s, 3H, acetyl-$CH_3$).

2-Acetoxy-4-maleimidobenzoylchlorid (Verbindung 6)

900 mg (3,27 mmol) 2-Acetoxy-4-maleimidobenzoesäure (Verbindung 5) wurden in einer Mischung aus 20 ml trockenem Toluol und 4 ml Thionylchlorid 2 h bei 120 °C gerührt. Dann wurde am Rotationsverdampfer zur Trockne eingeengt und im Hochvakuum getrocknet (Ausbeute 960 mg).

$^1$H-NMR (90MHz, $CDCl_3/D_6$-DMSO, TMS): 8.09 (d, 1H, $J_{5,6}$ = 9Hz, H-6); 7.46 (dd, 1H, $J_{3,5}$ = 2.5Hz, $J_{5,6}$ = 9Hz, H-5); 7.27 (d, 1H, $J_{3,5}$ = 2.5Hz, H-3); 7.14 (s, 2H, maleimido-H); 2.29 (s, 3H, acetyl-$CH_3$).

3-Maleimidopropionylchlorid (Verbindung 7)

508 mg (3,0 mmol) 3-Maleimidopropionsäure wurden in einer Mischung aus 25 ml trockenem Toluol und 2,2 ml Thionylchlorid 3 h bei 120 °C gerührt. Dann wurde am Rotationsverdampfer zur Trockne eingeengt und im Hochvakuum getrocknet (Ausbeute 560 mg).

Beispiel 1:

7-O-[4′-O-(4-Maleimidobenzoyl)-α-L-rhodosaminyl]-β-rhodomycinon (Verbindung 8)

29 mg (0,053 mmol) 7-O-(α-L-Rhodosaminyl)-β-rhodomycinon ("β″ Rhodomycin-I", Brockmann et al., Tetrahedron Letters 1969, 415)
wurden in 10 ml Chloroform gelöst und mit 10 ml gesättigter Natriumhydrogencarbonatlösung versetzt. Unter Rühren wurde eine Lösung von 18 mg (0,076 mmol) 4-Maleimidobenzoylchlorid (Verbindung 2) in 3 ml Chloroform zugesetzt und bei Raumtemperatur 16 h im Dunkeln gerührt. Danach wurde die organische Phase abgetrennt, am Rotationsverdampfer eingeengt und der Rückstand im Hochvakuum getrocknet (Ausbeute ca. 40 mg).

$^1$H-NMR (400MHz, $CDCl_3$, TMS): 13.61 (bs, 1H, OH-11); 12.87 (bs, 1H, OH-6); 12.10 (bs, 1H, OH-4); 8.32 (d, 2H, J = 8.8Hz, Phenylen: H-2, H-6); 7.88 (dd, 1H, $J_{1,2}$ = 7.5Hz, $J_{1,3}$ = 1.0Hz, H-1); 7.71 (t, 1H, H-2); 7.53 (dd, 2H, J = 8.7Hz, Phenylen: H-3, H-5); 7.33 (dd, 1H, $J_{1,3}$ = 1.0Hz, $J_{2,3}$ = 7.4Hz, H-3); 6.88 (s, 2H, maleimido-H); 5.67 (d, 1H, J = 3.3Hz, H-1′); 5.57 (s, 1H, H-4′); 5.18 (m, 1H, H-7); 4.93 (s, 1H, H-10); 4.33 (q, 1H, H-5′); 2.36 (bs, $N(CH_3)_2$); 2.4-2.0 (m, H-3′, $H_2$-2′, $H_2$ 8); 1.88 (m, 1H, H-13β); 1.78 (m, 1H, H-13α); 1.23 (d, 3H, $H_3$-6′); 1.13 (t, 3H, $H_3$-14).

Beispiel 2:

7-O-[4′-O-(2-Chlor-4-maleimidobenzoyl)-α-L-rhodosaminyl]-β-rhodomycinon [Verbindung 9)

31,5 mg (0,058 mmol) β-Rhodomycin-I und 45 mg (0,17 mmol) 2-Chlor-4-maleimidobenzoylchlorid (Verbindung 4) wurden in Analogie zu Beispiel 1 umgesetzt und aufgearbeitet.

Beispiel 3:

7-O-[4′-O-(2-Acetoxy-4-maleimidobenzoyl)-α-L-rhodosaminyl]-β-rhodomycinon (Verbindung 10)

31,5 mg (0,058 mmol) β-Rhodomycin-I und 48 mg (0,16 mmol) 2-Acetoxy-4-maleimidobenzoylchlorid (Verbindung 6) wurden in Analogie zu Beispiel 1 umgeserzt und aufgearbeitet.

Beispiel 4:

7-O-[4′-O-(3-Maleimidopropionyl)-α-L-rhodosaminyl]-βrhodomycinon [Verbindung 11]

31,5 mg (0,058 mmol) β-Rhodomycin-I und 30 mg (0,16 mmol) 3-Maleimidopropionylchlorid (Verbindung 7) wurden in Analogie zu Beispiel 1 umgesetzt und aufgearbeitet.

Beispiel 5:

4″-O-(4-Maleimidobenzoyl)-cytorhodin-S (Verbindung 12)

190 mg (0,20 mmol) Cytorhodin-S (Huber et al., ZA Patent Nr. 84/5538) wurden in 60 ml Chloroform gelöst und mit 20 ml gesättigter Natriumhydrogencarbonatlösung versetzt. Unter Rühren wurde eine Lösung von 53 mg (0,225 mmol) 4-Maleimidobenzoylchlorid (Verbindung 2) in 3 ml Chloroform zugesetzt und bei Raumtemperatur 2 h im Dunkeln gerührt. Danach wurde die organische Phase abgetrennt, am Rotationsverdampfer eingeengt und der Rückstand im Hochvakuum getrocknet (Ausbeute ca 200 mg).

$^1$H-NMR (400MHz, $CDCl_3$, TMS): 13.77 (bs, 1H, OH-11); 12.86 (bs, 1H, OH-6); 12.11 (bs, 1H, OH-4); 8.17 (d, 2H, J = 8.7Hz, Phenylen: H-2, H-6); 7.90 (d, 1H, J = 7.6Hz, H-1); 7.71 (t, 1H, H-2); 7.50 (d, 2H, J = 8.7Hz, Phenylen H-3, H-5); 7.31 (dd, 1H, $J_{1,3}$ = 1.0Hz, $J_{2,3}$ = 8.4Hz, H-3); 6.88 (s, 2H, maleimido-H); ......; 2.21 (bs, 2x $N(CH_3)_2$); .......; 1.36 (d, 3H), 1.30 (d, 3H), 1.22 (d, 3H) und 1.17 (d, 3H) (4x$H_3$-6′); 1.12 (t, 3H, $H_3$-14).

Stabilitätsuntersuchung der gespacerten Anthracycline (Verbindungen 8-11)

Die jeweilige Verbindung wurde bei einer Konzentration von 1 mg/ml in Acetonitril gelöst und mit Phosphat-gepufferter Kochsalzlösung pH 7.2 im Volumenverhältnis 1:100 verdünnt, wobei eine Konzentration von jeweils 10 µg/ml erhalten wurde. Diese Lösungen wurden jeweils bei 37 °C im Dunkeln inkubiert und 20 µl-Aliquote wurden nach definierten Zeiten mittels der HPLC-Technik untersucht. Die Stabilitäten dieser Verbindungen sind in Fig. 1 graphisch dargestellt.

Hydrolyse von Verbindung 10

Verbindung 10 wurde, wie vorstehend beschrieben, unrersucht und mittels HPLC-Technik analysiert. Dabei wurde gefunden, daß sich die Verbindung unter den gegebenen Bedingungen hydrolytisch zersetzt, wobei die Hydrolyse sowohl direkt als auch über ein nichr näher charakterisiertes Zwischenprodukt zur Freisetzung von 7-O-(α-L-Rhodosaminyl)-β-rhodomycinon (β-Rhodomycin-I) führt (Fig. 2).

Hydrolyse von Verbindung 11

Verbindung 11 wurde analog zur Verbindung 10 hydrolysiert, wobei analoges Hydrolyseverhalten festgestellt wurde (Fig. 3).

Hydrolyse von Verbindung 8

Verbindung 8 wurde analog zur Verbindung 10 hydrolysiert, wobei ebenfalls analoges Hydrolyseverhalten festgestellt wurde (Fig. 4).

Hydrolyse von Verbindung 12

Verbindung 12 wurde analog zu Verbindung 10 hydrolysiert, wobei jedoch ein pH-Wert von 7,4 verwendet wurde. Auch hier wurde ein analoges Hydrolyseverhalten festgestellt (Fig. 5).

Beispiel 6:

Kopplung von Verbindung 12 an anti-CEA-MAK 431/26 zu Verbindung 13

Eine Lösung des monoklonalen Antikörpers MAK 431/26 (EP 0160897 vom 07.05.84 und die äquivalente US-Anmeldung Ser.No. 729,578 - Bosslet et al.) (330 mg entspre chend 10,5 ml einer 3,12-proz. Lösung, bestehend aus 10 mM Natriumphosphat, 75 mM Kochsalz und 30 g/Liter Sorbit, pH 7.2) wurde mit 30,8 mg Dithiothreitol 30 min bei Raumtemperatur inkubiert. Der reduzierte Antikörper wurde mittels Gelfiltration über Sephadex G25 in physiologischer Kochsalzlösung pH 6.0 in einem resultierenden Elutionsvolumen von 30 ml isoliert. Diese Lösung wurde mit 6 ml Dimethylformamid, welches zuvor auf pH 6.0 eingestellt worden war, verdünnt und mir einer Lösung von 74 mg (64,9 µmol) 4″-O-(4-Maleimidobenzoyl)-cytorhodin-S (Verbindung 12), gelost in 2 ml Dimethylformamid, versetzt und bei Raumtemperatur im Dunkeln inkubiert. Nach 1 h wurde gebildetes Präzipitat bei 4000 g abzentrifugiert und der Überstand über einen 0,45 µ-Filter gepreßt. Das Filtrat wurde über eine Sephadex G25-Säule (80 ml Gel) in 0,85 % Kochsalzlösung pH 7.2 filtriert, wobei das Konjugat von ungebundenem Zytostatikum abgetrennt wurde. Der das Konjugat enthaltende Elutionspeak (120 ml) wurde gesammelt. Das Konjugat wurde durch Zusatz einer bei Raumtemperatur gesättigten Ammoniumsulfat-Lösung (120 ml) ausgefällt, das Präzipitat abzentrifugiert, gegen physiologische Kochsalzlösung von pH 7.2 dialysiert und sterilfiltriert, wobei 13 ml Konjugatlösung erhalten wurden. Der Kopplungsgrad wurde durch Extinktionsmessung (Fig. 6) und über Bestimmung des Proteingehalts der Lösung nach Kjeldahl zu 7,6 Mol Cytorhodin-S je Mol MAK 431/26 bestimmt. Die KonjugatLösung wurde bei -18 °C präserviert.

Beispiel 7:

Kopplung von Verbindung 12 an anti-Pankreas-CA-MAK 494/32 zu Verbindung 14

Die Kopplung erfolgte analog Beispiel 6 mit analogen Mengen. MAK 494/32 wurde von Bosslet et al. (EP 86111708 vom 02.09.85 und die äquivalente US-Anmeldung Ser.No. 901,873) beschrieben. Der Kopplungsgrad (Extinktions messung s. Fig. 7) wurde zu 7,1 Mol Cytorhodin-S je Mol MAK 494/32 bestimmt.

Beispiel 8:

Kopplung von Verbindung 8 an polyklonales humanes IgG (Tetagam$^R$)zu Verbindung 15

Die Kopplung erfolgte analog Beispiel 6 mit 5 mg (6,7 µmol) 7-O-[4′-O-(4-Maleimidobenzoyl)-α-L-rhodosaminyl]-β-rhodomycinon (Verbindung 8) und 34 mg Tetagam$^R$ in Form einer 2-proz. Lösung. Der Kopplungserfolg wurde durch Extinktionsmessung analog Fig. 6 und 7 bestätigt.

Beispiel 9:

Kopplung von Verbindung 8 an anti-Pankreas-CA-MAK 494/32 zu Verbindung 16

Die Kopplung erfolgte analog Beispiel 7 bzw. 6 mit 7,7 mg (10 µmol) 7-O-(4′-O-(4-Maleimidobenzoyl)-α-L-rhodosaminyl]-β-rhodomycinon (Verbindung 8) und 35 mg MAK 494/32. Der Kopplungserfolg wurde durch Extinktionsmessung analog Fig. 6 und 7 bestätigt.

Beispiel 10:

7-O-[4'-O-(4-Maleimidobenzoyl)-α-L-rhodosaminyl ] -daunomycinon (Verbindung 17)

20,5 mg (0,037 mmol) N,N-Dimethyldaunorubicin (Tong et al., J. Med. Chem. (1979), 22, 912) wurden in Analogie zu Beispiel 1 mit 11,8 mg (0,05 mmol) 4-Maleimidobenzoylchlorid (Verbindung 2) umgesetzt und aufgearbeitet (Ausbeute ca. 27 mg).

$^1$H-NMR (400 MHz, $CDCl_3$, TMS): 14.01 (s, 1H, OH-6); 13.28 (s, 1H, OH-11); 8.21 (d, 2H, J = 8.3 Hz, Phenylen: H-2, H-6); 8.04 (d, 1H, $J_{1,2}$ = 7,6 Hz, H-1); 7,78 (t, 1H, H-2); 7.52 (d, 2H, J = 8.3 Hz, Phenylen: H-3, H-5); 7.39 (d, 1H, $J_{2,3}$ = 8.4 Hz, H-3); 6.89 (s, 2H, maleimido-H); 5.70 (bs, 1H, H-1'); 5.53 (bs, 1H, H-4'); 5.34 (m, 1H, H-7); 4.27 (q, 1H, H-5'); 4.09 (s, 3H, $OCH_3$); 3.25 (d, 1H, H-10β); 2.98 (d, 1H, H-10α); 2.44 (s, 3H, $H_3$-14); 2.26 (s, 6H, $N(CH_3)_2$); 1.21 (d, 3H, $H_3$-6').

Beispiel 11:

Kopplung von Verbindung 17 an anti-Pankreas-CA-MAK 494/32 zu Verbindung 18

Die Kopplung erfolgt analog Beispiel 7 bzw. 6 mit 6,4 mg (8,5 µmol) 7-O-[4'-O-(4-Maleimidobenzoyl)-α-L-rhodosaminyl ]daunomycinon (Verbindung 17) und 30 mg MAK 494/32. Der Kopplungserfolg wurde durch Extinktionsmessung analog Fig. 6 und 7 bestätigt.

Spezifitäts-Nachweis der Cytorhodin-S-Konjugate 13 und 14 in vivo

Cytorhodin-S-anti-CEA-MAK 431/26 (Verbindung 13) und Cytorhodin-S-anti-Pankreas-CA-MAK 494/32 (Verbindung 14) wurden im Xenograft-Nacktmaus-Modell auf ihre in vivo-Bindung an CO-WI-Tumoren untersucht (Verteilung 3 Tage nach Applikation). Als Kontrolle diente polyklonales Maus-Immunglobulin.

Hierzu wurden die Konjugate und die Kontrolle jeweils in an sich bekannter Weise (IODO-GEN$^R$-Methode, Oxidationsmittel: 1,3,4,6-Tetrachloro-3a,6a-diphenylglycoluril; P.J. Fraker, J.C. Speck (1978), Biochem. Biophys. Res. Commun. 80, 849-857) mit radioaktivem Jod (Konjugate: $^{131}$I, Kontrolle: $^{125}$I) markiert und in Gruppen von jeweils 3 Nacktmäusen i.v. appliziert. Nach 3 Tagen wurden die Tiere getötet und die einzelnen Organe auf enthaltenes $^{125}$ I und $^{131}$ I vermessen.

Für beide Konjugate wurde die spezifische Anreicherung in CO-WI-Tumoren durch die in den Organen und im Tumor gefundene Radioaktivitat (ausgedrückt in ng Konjugate/g Organ bzw. Tumor, s. Fig. 8) und nach Umrechnung in sogenannte Lokalisierungsindices (Methode von V. Moshakis, M.J. Bailey, M.G. Ormerod, J.H. Westwood, A.M. Neville (1981), Br. J. Cancer 43, 575-581; s. Fig. 9, Definition des L.I.-Wertes s. dort) bestätigt.

Spezifitäts-Nachweis der MAK 494/32-Konjugate 14 und 16

β-Rhodomycin-I-MAK 494/32 (Verbindung 16), Cytorhodin-S-MAK 494/32 (Verbindung 14) und MAK 494/32 (ungekoppelt) wurden im Xenograft-Nacktmaus-Modell in Analogie zum vorstehenden Verfahren auf ihre in vivo-Bindung an CO-WI-Tumoren untersucht (Verteilung 3 Tage nach Applikation). Die Lokalisationsindices sind in Fig. 10 zusammengefaßt.

In den beigefügten Zeichnungen sind die mit den erfindungsgemäßen Verbindungen erhaltenen Ergebnisse graphisch dargestellt.

Im einzelnen zeigen:

Fig. 1:

die Stabilität von drei 7-O-[4′ O-(4-Maleimidobenzoyl]-α-L-rhodosaminyl]-β-rhodomycinone bei Variation des Substituenten Z der Phenylengruppe des Spacers (siehe Formel IV) gegenüber 7-O-[4′-O-(3-Maleimidopropionyl)-α-L-rhodosaminyl]-β-rhodomycinon (Verbindung 11) als Standard in Phosphat-gepufferter Kochsalzlösung, pH 7.2 bei 37° C, ermittelt durch HPLC-Analytik.

Fig. 2:

die Hydrolyse von Verbindung 10

Fig. 3:

die Hydrolyse von Verbindung 11

Fig. 4:

die Hydrolyse von Verbindung 8

Fig. 5:

die Hydrolyse von Verbindung 12

Fig. 6:

den Extinktionsverlauf der in Beispiel 6 dargestellten Konjugatlösung - nach 50facher Verdünnung - in Abhängigkeit von der Wellenlänge

Fig 7:

den Extinktionsverlauf der in Beispiel 7 dargestellten Konjugatlösung - nach 50facher Verdünnung - in Abhängigkeit von der Wellenlänge

Fig. 8:

die Anreicherung der Konjugate 13 (A) und 14 (B) in den Organen Xenograft-tragender Nacktmäuse, ausgedrückt in ng Konjugat pro g Organ bzw. Tumor, nach 3 Tagen. Bezeichnung der Organe: 1 Blut, 2 Blase, 3 Herz, 4 Darm, 5 Niere links, 6 Niere rechts, 7 Leber, 8 Milz, 9 Lunge, 10 Kopf, 11 Restkörper, 12 Magen, 13 Tumor, 14 Muskel. Die genannten Werte sind Mittelwerte aus jeweils 3 Einzeltieren.

Fig. 9:

die Lokalisierungsindices L.I. der Konjugate 13 (A) und 14 (B) in den einzelnen Organen, nach 3 Tagen.

$$L.I. = \frac{cpm\ (^{131}I,\ Organ)}{cpm\ (^{131}I,\ Blut)} : \frac{cpm\ (^{125}I,\ Organ)}{cpm\ (^{125}I,\ Blut)}$$

Nach V. Moshakis et al., Br. J. Cancer (1981), 43, 575-581. Bezeichnung der Organe: s. Ausführungen zu Fig. 8, Tumor = Organ Nr. 13. Die angegebenen L.I.-Werte sind Mittelwerte aus jeweils 3 Tieren.

Fig. 10:

die Tumoranreicherung der Konjugate 16 (B) und 14 (C) im Vergleich zu nicht konjugiertem MAK 494/32 (A) ausgedrückt in L.I.-Werten für verschiedene Organe einschließlich Tumor. Definition der Organe: siehe Ausführungen zu Fig. 8, Tumor = Organ Nr.13; L.I.-Werte = Mittelwerte aus Doppelbestimmungen.

**Ansprüche**

1. Neue zytostatisch wirksame Anthracyclinderivate der allgemeinen Formel I, welche ggf. als Salz einer anorganischen oder organischen Säure vorliegen,

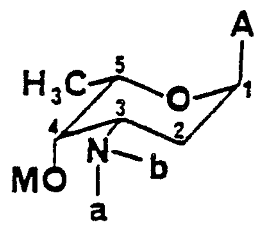

in der die Reste folgende Bedeutung haben:
A ist ein Anthracyclinon der allgemeinen Formel II oder III,

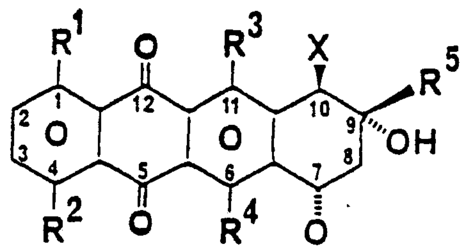

II

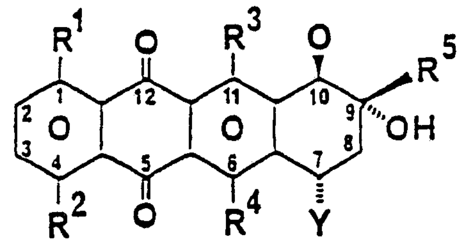

III

M ist eine Struktur der allgemeinen Formel IV oder V,

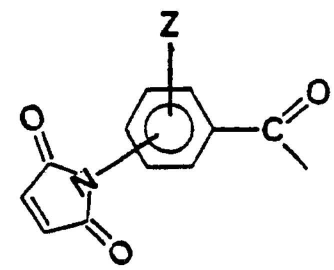

IV

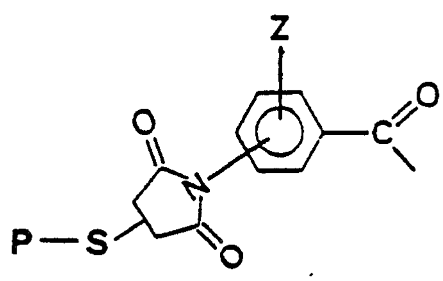

V

und a und b sind gleichartige oder verschiedenartige substituierte oder unsubsrituierte Alkylreste aliphatischer oder benzylaromatischer Natur oder a und b bilden, zusammen mit dem N-Atom, einen gegebenenfalls substituierten heterozyklischen Ring, vorzugsweise eine Morpholino- oder Cyanomorpholino-Struktur, wobei in den Formeln II und III

$R^1$ Wasserstoff oder eine Hydroxygruppe,

$R^2$ Wasserstoff oder eine Hydroxy- oder eine Methoxygruppe,

$R^3$ Wasserstoff oder eine Hydroxygruppe,

$R^4$ Wasserstoff oder eine Hydroxygruppe

$R^5$ $CH_2CH_3$, $COCH_3$ oder eine Struktur der Formel $COCH_2OCOR$, wobei R zusammen mit der Carbonylgruppe die Acylfunktion einer einbasigen oder einer $\alpha,\omega$-zweibasigen aliphatischen Carbonsäure oder einer Aminosäure darstellt,

X Wasserstoff, eine Hydroxy- oder eine Methoxycarbonylgruppe oder ein Rest der Formel VI

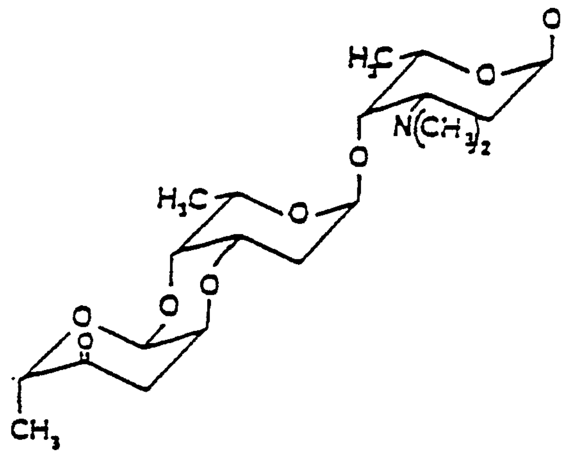

VI

und Y ein Rest der Formel VI ist, wobei in den Formeln IV und V der Rest Z Wasserstoff oder eine Methyl-, Hydroxy-, Methoxy-, Acetoxy-, Nitro- oder Cyanogruppe oder ein Chlor- oder Bromatom sein kann und wobei in der Formel V der Rest P ein Protein- oder Peptid-Rest, insbesondere ein Immunglobulin oder Immunglobulin-Rest, bevorzugt ein monoklonaler Antikörper oder ein Fab-, Fab′- oder ein F(ab′)$_2$ Fragment sein kann, wobei P ggf. weitere Anthracyclinderivate der beanspruchten gekoppelten Art tragen kann, oder P kann insbesondere auch ein Hormon-, Lectin- oder Wachstumsfaktorrest sein.

2. Anthracyclinderivate nach Anspruch 1,
**dadurch gekennzeichnet**
, daß A ein Anthracyclinon der Formel II und M eine Struktur der Formel IV ist.

3. Anthracyclinderivate nach Anspruch 1,
**dadurch gekennzeichnet**
, daß A ein Anthracyclinon der Formel II und M eine Struktur der Formel V ist.

4. Anthracyclinderivate nach Anspruch 1,
**dadurch gekennzeichnet**
, daß A ein Anthracyclinon der Formel III und M eine Struktur der Formel IV ist.

5. Anthracyclinderivate nach Anspruch 1,
**dadurch gekennzeichnet**
, daß A ein Anthracyclinon der Formel III und M eine Struktur der Formel V ist.

6. Anthracyclinderivate nach Anspruch 1,
**dadurch gekennzeichnet**
, daß A ein Anthracyclinon der Formel II und $R^1$ = OH, $R^2$ = $R^3$ = $R^4$ = OH, $R^5$ = $CH_2CH_3$ und X = OH ist.

7. Anthracyclinderivate nach Anspruch 1,
**dadurch gekennzeichnet**
, daß A ein Anthracyclinon der Formel II und $R^1$=H, $R^2$=$OCH_3$, $R^3$=$R^4$=OH, $R^5$=$COCH_3$ oder eine Struktur der Formel $COCH_2OCOR$ ist, wobei R zusammen mit der Carbonylgruppe die Acylfunktion einer einbasigen oder einer $\alpha\omega$-zweibasigen aliphatischen Carbonsäure oder einer Aminosäure darstellt, und X=H ist.

8. Anthracyclinderivate nach Anspruch 1,
**dadurch gekennzeichnet**
, daß A ein Anthracyclinon der Formel II und $R^1$=H, $R^2$=$R^3$=$R^4$=OH, $R^5$=$CH_2CH_3$ und X=OH ist.

9. Anthracyclinderivate nach Anspruch 1,
**dadurch gekennzeichnet**, daß A ein Anthracyclinon der Formel III und $R^1$ = OH, $R^2$=$R^3$=$R^4$=OH, $R^5$=$CH_2CH_3$ und Y ein Rest der Formel VI ist.

10. Anthracyclinderivate nach Anspruch 1,
**dadurch gekennzeichnet**
, daß A ein Anthracyclinon der Formel III ist und $R^1$=H, $R^2$=$R^3$=$R^4$=OH, $R^5$=$CH_2CH_3$ und Y ein Rest der Formel VI ist.

11. Anthracyclinderivate nach Anspruch 1,
**dadurch gekennzeichnet**
, daß M eine Struktur der Formel V ist und P den Rest eines monoklonalen Antikörpers darstellt, welcher gegen einen Tumor gerichtet ist.

12. Anthracyclinderivate nach Anspruch 1,
**dadurch gekennzeichnet,**
daß M eine Struktur der Formel V ist und P den Rest eines monoklonalen Antikörpers darstellt, welcher gegen das carcinoembryonale Antigen gerichtet ist.

13. Anthracyclinderivate nach Anspruch 1,
**dadurch gekennzeichnet,**
daß M eine Struktur der Formel V ist und P den Rest eines monoklonalen Antikörpers darstellt, welcher gegen Pankreas-Tumoren gerichtet ist.

14. Verfahren zur Herstellung von Anthracyclinderivaten nach Anspruch 1,
**dadurch gekennzeichnet,**
daß man eine Verbindung der Formel I, in welcher M Wasserstoff ist, mit einem ggf. im aromatischen Ring durch eine Methyl-, Hydroxy-, Methoxy-, Acetoxy-, Nitro- oder Cyanogruppe oder ein Chlor- oder Bromatom substituierten reaktionsfähigen Maleimidobenzoesäurederivat in einem Zweiphasensystem, bestehend aus einem mit Wasser nicht mischbaren Lösungsmittel und einer wäßrigen Phase, in welcher eine geeignete Base enthalten ist, zu einer Verbindung der Formel I umsetzt, in welcher M eine Struktur der Formel IV darstellt und aus der organischen Phase die Verbindung der Formel I isoliert, diese gegebenenfalls mit einem SH-Gruppen tragenden Protein oder Peptid oder Peptid-Rest, insbesondere einem SH-Gruppen tragenden Immunglobulin oder Immunglobulin-Rest, bevorzugt einem SH-Gruppen tragenden monoklonalen Antikörper oder einem Fab- oder Fab′ oder einem F(ab′)$_2$-Fragment oder einem SH-Gruppen tragenden Hormon, Lectin oder Wachstumsfaktor in einem gepufferten wäßrigen Medium zu einer Verbindung der Formel I umsetzt, in welcher M eine Struktur der Formel V aufweist und die so gewonnene Verbindung der Formel I in üblicher Weise isoliert oder aufarbeitet.

15. Verfahren nach Anspruch 14,
**dadurch gekennzeichnet,**
daß die SH-Gruppen im Molekül bereits vorhanden sind oder die SH-Gruppen durch Reduktion vorhandener S-S-Bindungen erzeugt werden oder durch sonstige chemische Reaktionen eingeführt oder erzeugt werden.

16. Verfahren nach Anspruch 14,
**dadurch gekennzeichnet,**
daß als reaktionsfähiges Maleimidobenzoesäurederivat das Säurechlorid oder das Säurebromid eingesetzt wird.

17. Verfahren nach Anspruch 14,
**dadurch gekennzeichnet,**
daß für die Bildung des Anthracyclinkonjugates ein physiologisches Puffer-System, welches gegebenenfalls bis zu 30 Volumenprozent Dimethylformamid oder Dimethylsulfoxid enthalten kann, verwendet wird.

18. Verwendung eines der Anthracyclinderivate nach Anspruch 1 in einem Arzneimittel oder Diagnostikum.

Claims for the following Contracting States: SP; GR.

1. Verfahren zur Herstellung neuer zytostatisch wirksamer Anthracyclinderivate der allgemeinen Formel I, welche ggf. als Salz einer anorganischen oder organischen Säure vorliegen.

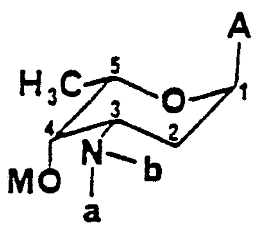

I

in der die Reste folgende Bedeutung haben:
A ist ein Anthracyclinon der allgemeinen Formel II oder III,

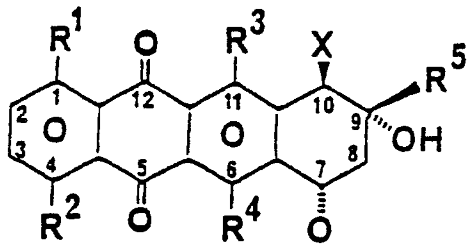

II

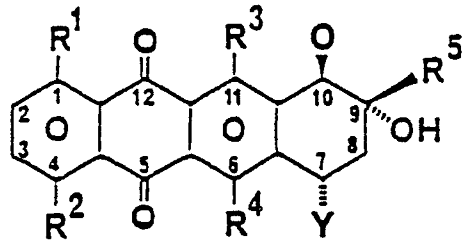

III

M ist eine Struktur der allgemeinen Formel IV oder V,

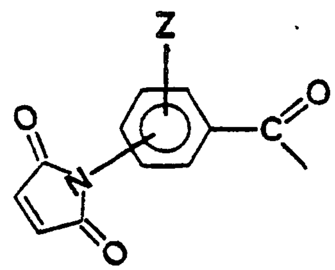

IV

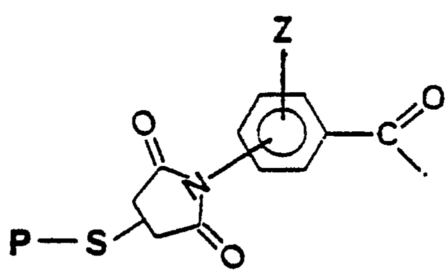

V

und a und b sind gleichartige oder verschiedenartige substituierte oder unsubstituierte Alkylreste aliphatischer oder benzylaromatischer Natur oder a und b bilden, zusammen mit dem N-Atom, einen gegebenenfalls substituierten heterozyklischen Ring, vorzugsweise eine Morpholino- oder Cyanomorpholino-Struktur, wobei in den Formeln II und III

$R^1$ Wasserstoff oder eine Hydroxygruppe,

$R^2$ Wasserstoff oder eine Hydroxy- oder eine Methoxygruppe,

$R^3$ Wasserstoff oder eine Hydroxygruppe,

$R^4$ Wasserstoff oder eine Hydroxygruppe

$R^5$ $CH_2CH_3$, $COCH_3$ oder eine Struktur der Formel $COCH_2OCOR$, wobei R zusammen mit der Carbonylgruppe die Acylfunktion einer einbasigen oder einer $\alpha,\omega$-zweibasigen aliphatischen Carbonsäure oder einer Aminosäure darstellt,

X Wasserstoff, eine Hydroxy- oder eine Methoxycarbonylgruppe oder ein Rest der Formel VI

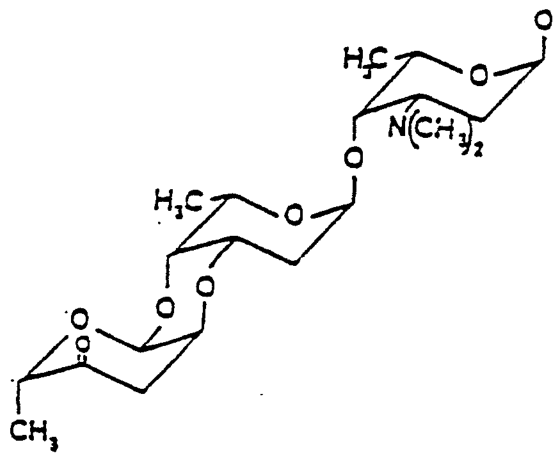

VI

und Y ein Rest der Formel VI ist,

wobei in den Formeln IV und V der Rest Z Wasserstoff oder eine Methyl-, Hydroxy-, Methoxy-, Acetoxy-, Nitro- oder Cyanogruppe oder ein Chlor- oder Bromatom sein kann und wobei in der Formel V der Rest P ein Protein- oder Peptid-Rest, insbesondere ein Immunglobulin oder Immunglobulin-Rest, bevorzugt ein monoklonaler Antikörper oder ein Fab-, Fab′- oder ein F(ab′)$_2$-Fragment sein kann, wobei P ggf. weitere Anthracyclinderivate der beanspruchten gekoppelten Art tragen kann, oder P insbesondere auch ein Hormon-, Lectin- oder Wachstumsfaktorrest sein kann,

**dadurch gekennzeichnet,**

daß man eine Verbindung der Formel I, in welcher M Wasserstoff ist, mit einem ggf. im aromatischen Ring durch eine Methyl-, Hydroxy-, Methoxy-, Acetoxy-, Nitro- oder Cyanogruppe oder ein Chlor- oder Bromatom substituierten reaktionsfähigen Maleimidobenzoesäurederivat in einem Zweiphasensystem, bestehend aus einem mit Wasser nicht mischbaren Lösungsmittel und einer wäßrigen Phase, in welcher eine geeignete Base enthalten ist, zu einer Verbindung der Formel I umsetzt, in welcher M eine Struktur der Formel IV darstellt und aus der organischen Phase die Verbindung der Formel I isoliert, diese gegebenenfalls mit einem SH-Gruppen tragenden Molekül P, wobei P die vorstehend angegebene Bedeutung hat, in einem gepufferten wäßrigen Medium zu einer Verbindung der Formel I umsetzt, in welcher M eine Struktur der Formel V aufweist und die so gewonnene Verbindung der Formel I in üblicher Weise isoliert oder aufarbeitet.

2. Verfahren nach Anspruch 1,

**dadurch gekennzeichnet,**

daß die SH-Gruppen im Rest P vorhanden sind.

3. Verfahren nach Anspruch 1,

**dadurch gekennzeichnet,**

daß die SH-Gruppen in P durch Reduktion vorhandener S-S-Bindungen erzeugt werden.

4. Verfahren nach Anspruch 1,

**dadurch gekennzeichnet,**

daß die SH-Gruppen in P durch chemische Reaktion eingeführt oder erzeugt werden.

5. Verfahren nach Anspruch 1,

**dadurch gekennzeichnet,**

daß als reaktionsfähiges Maleimidobenzoesäurederivat das Säurechlorid oder das Säurebromid eingesetzt wird.

6. Verfahren nach Anspruch 1,

**dadurch gekennzeichnet,**

daß für die Bildung des Anthracyclinkonjugates ein physiologisches Puffer-System, welches gegebenenfalls bis zu 30 Volumenprozent Dimethylformamid oder Dimethylsulfoxid enthalten kann, verwendet wird.

7. Verfahren zur Herstellung eines Arzneimittels oder Diagnostikums unter Verwendung eines Anthracyclinderivates, das nach Anspruch 1 erhalten wurde.

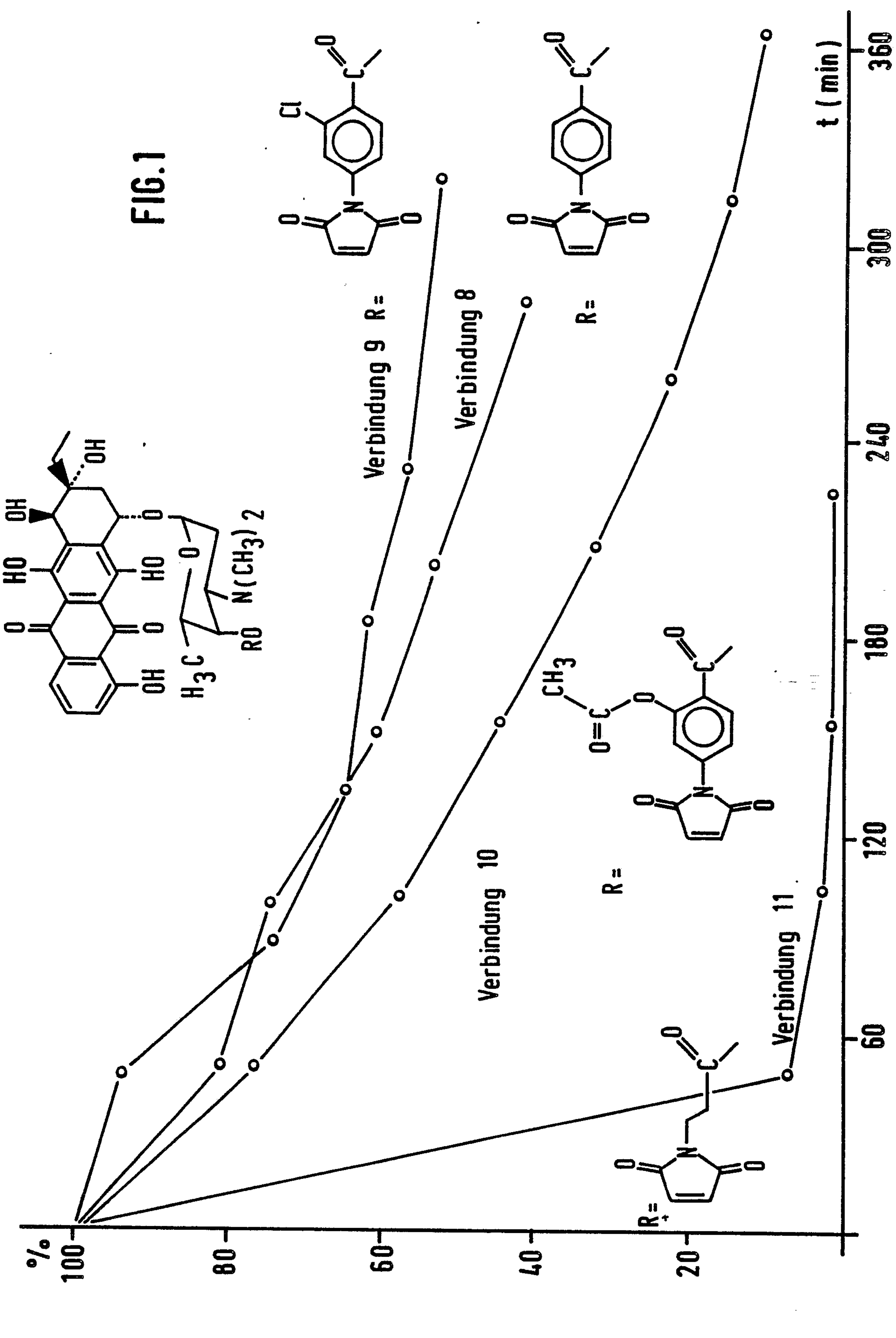
%
100
80
60
40
20
60
120
180
240
300
360
t (min)
Verbindung 9 R=
Verbindung 8
R=
Verbindung 10
R=
Verbindung 11
R=

FIG.1

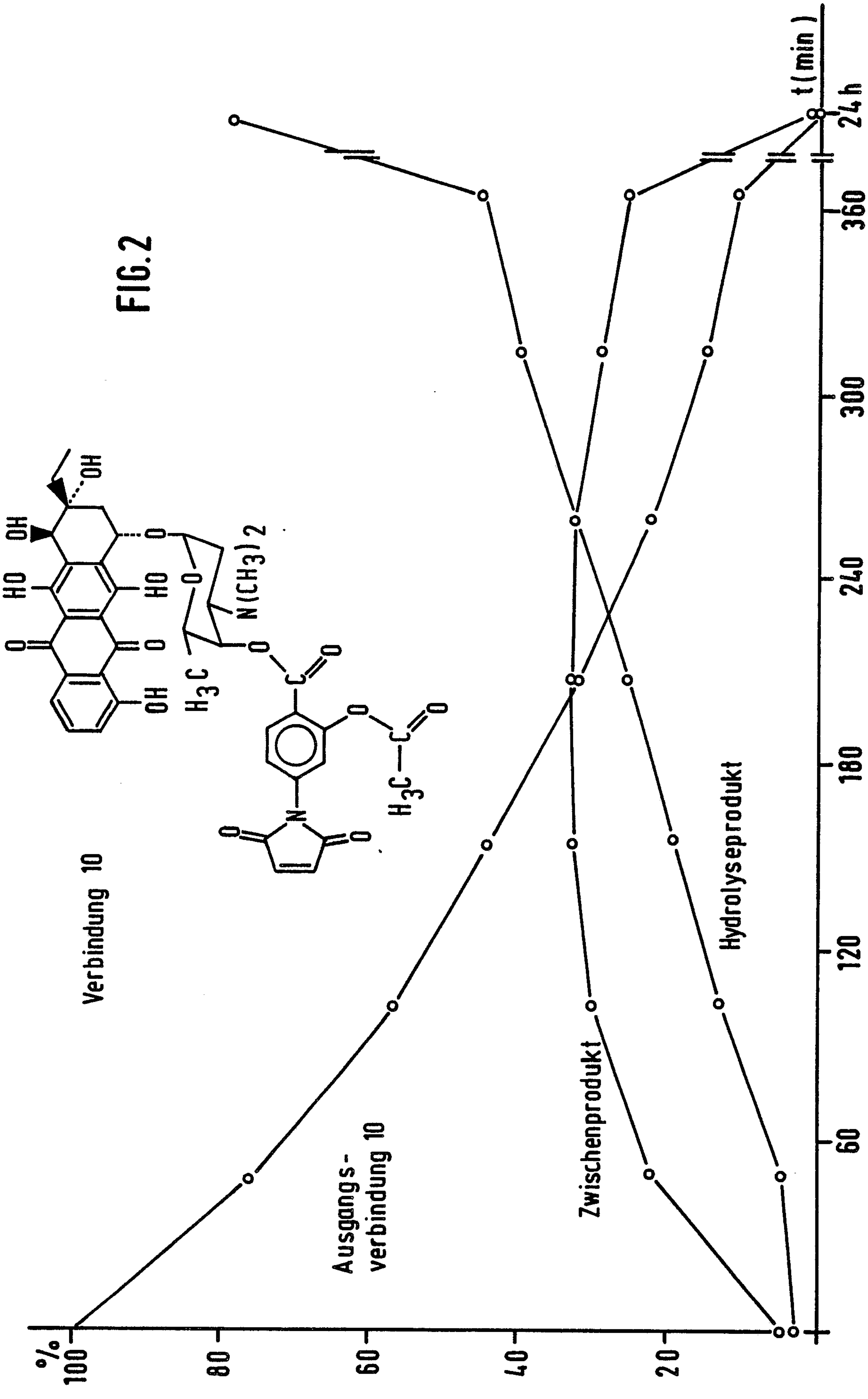
Verbindung 10
Ausgangs-
verbindung 10
Zwischenprodukt
Hydrolyseprodukt
%
100
80
60
40
20
60
120
180
240
300
360
24 h
t (min)

FIG.2

FIG. 3

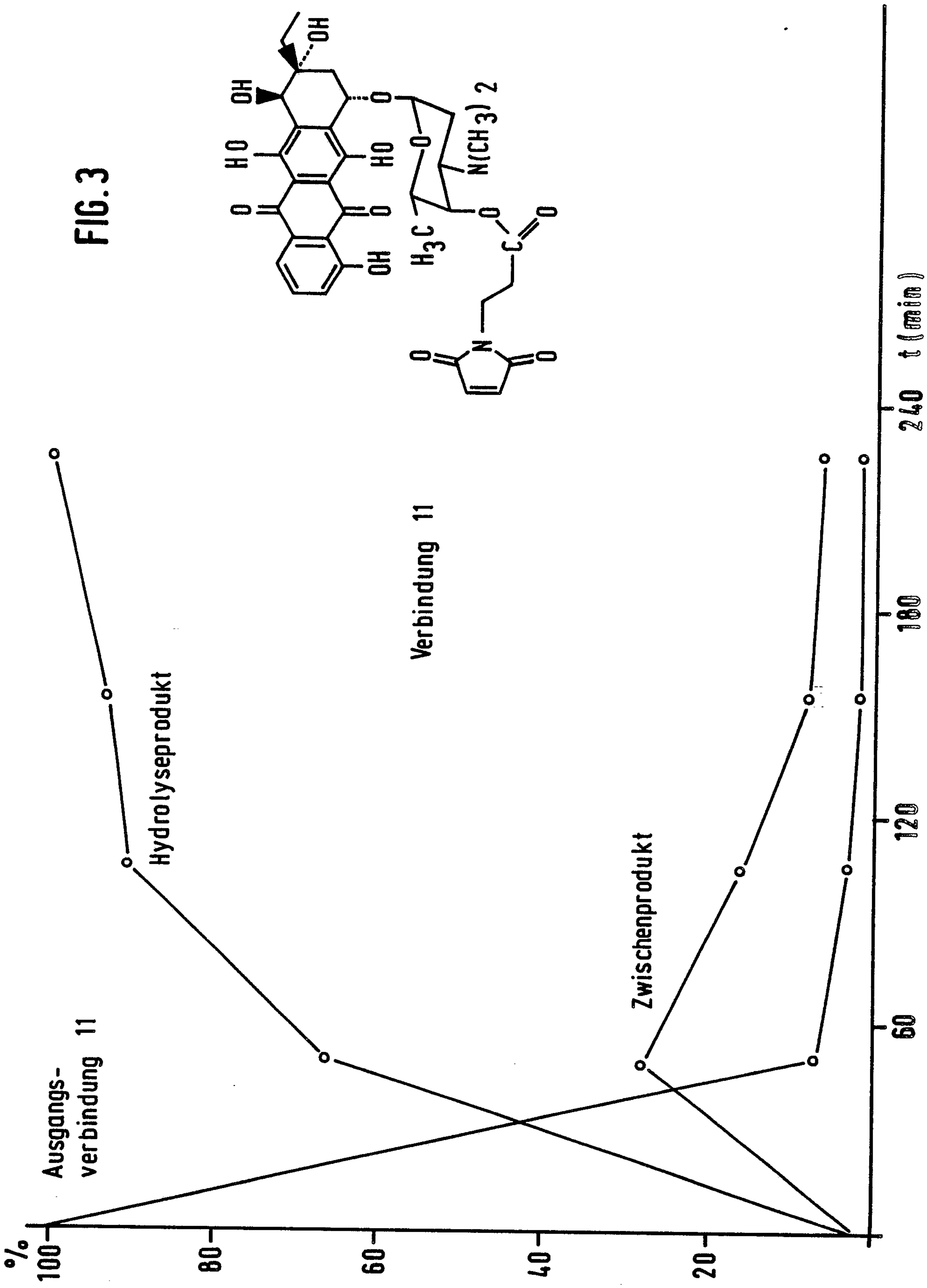
Verbindung 11
Ausgangs-
verbindung 11
Hydrolyseprodukt
Zwischenprodukt
%
100
80
60
40
20
60
120
180
240
t (min)

FIG. 4

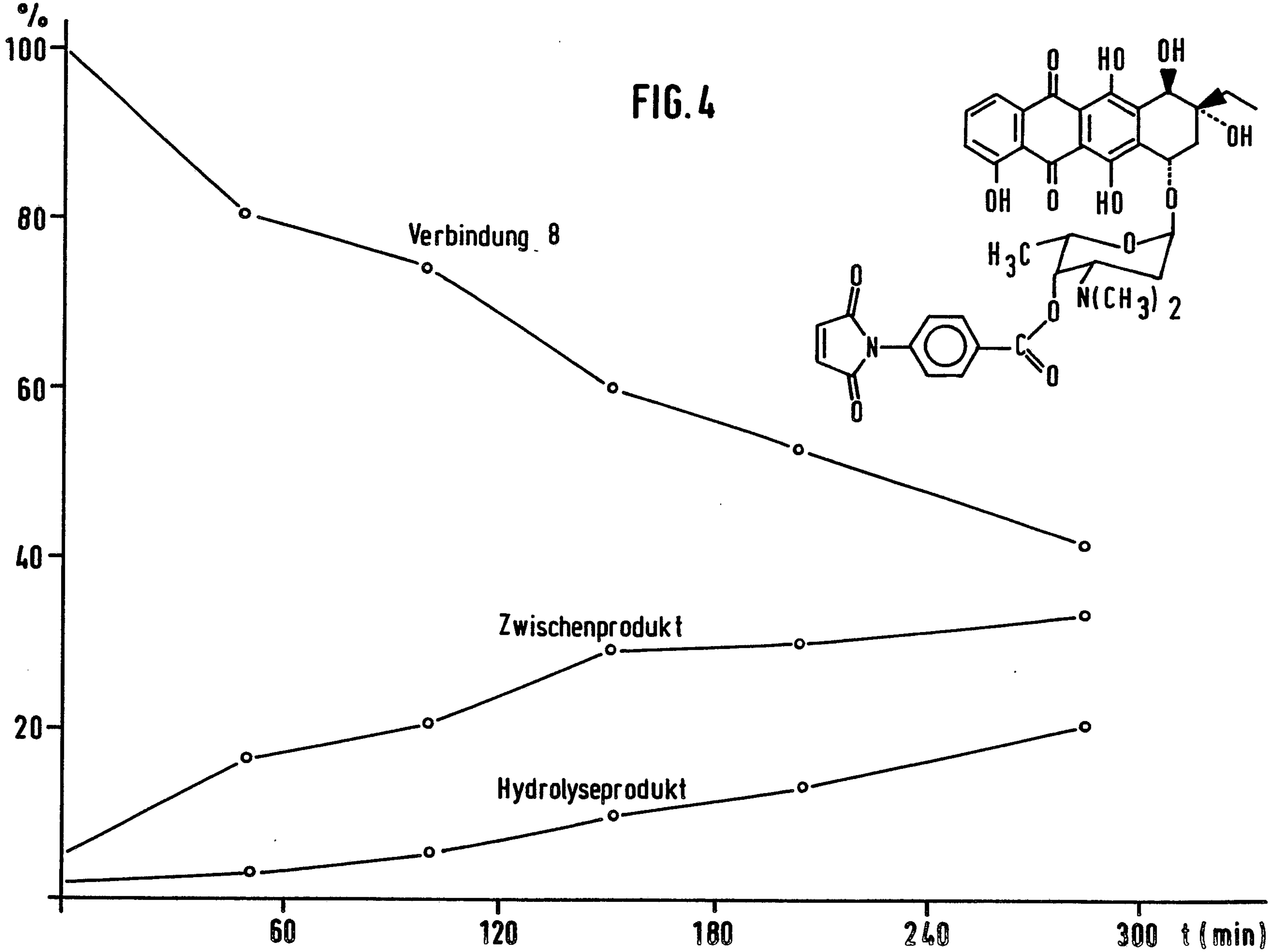
%
100
80
60
40
20
60
120
180
240
300 t (min)
Verbindung 8
Zwischenprodukt
Hydrolyseprodukt

FIG. 5

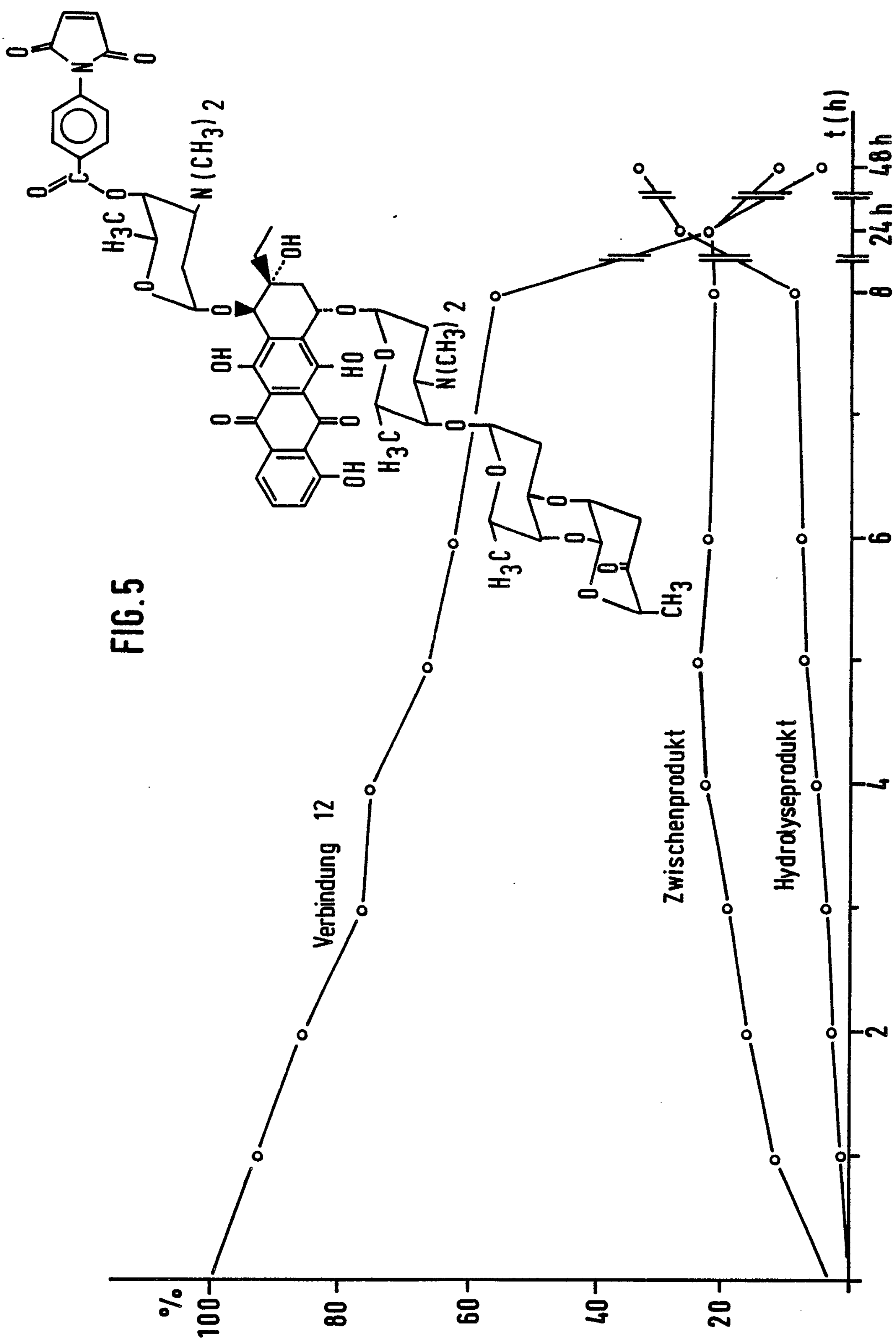
Verbindung 12
Zwischenprodukt
Hydrolyseprodukt
%
100
80
60
40
20
t (h)
2
4
6
8
24 h
48 h

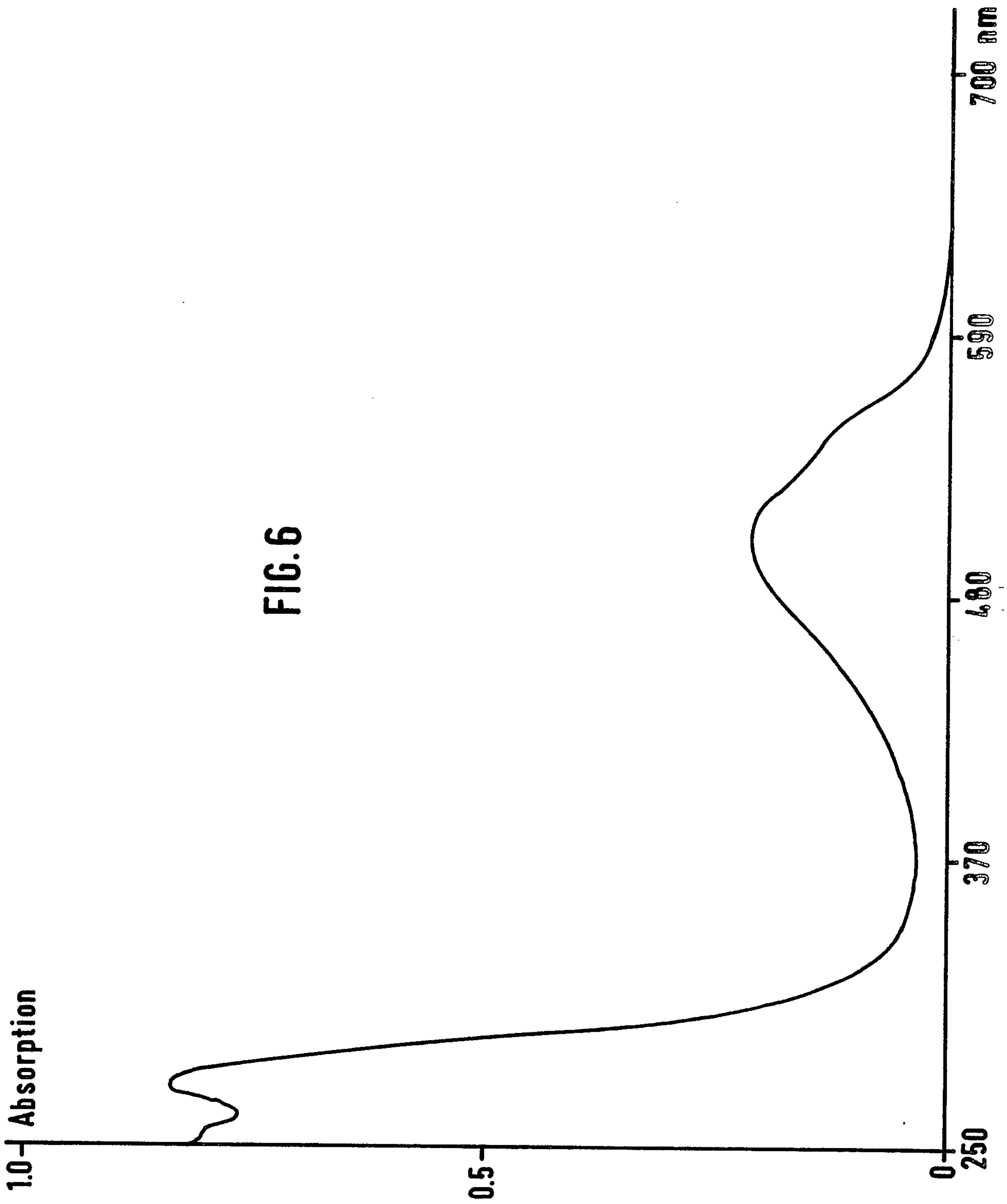


FIG. 6

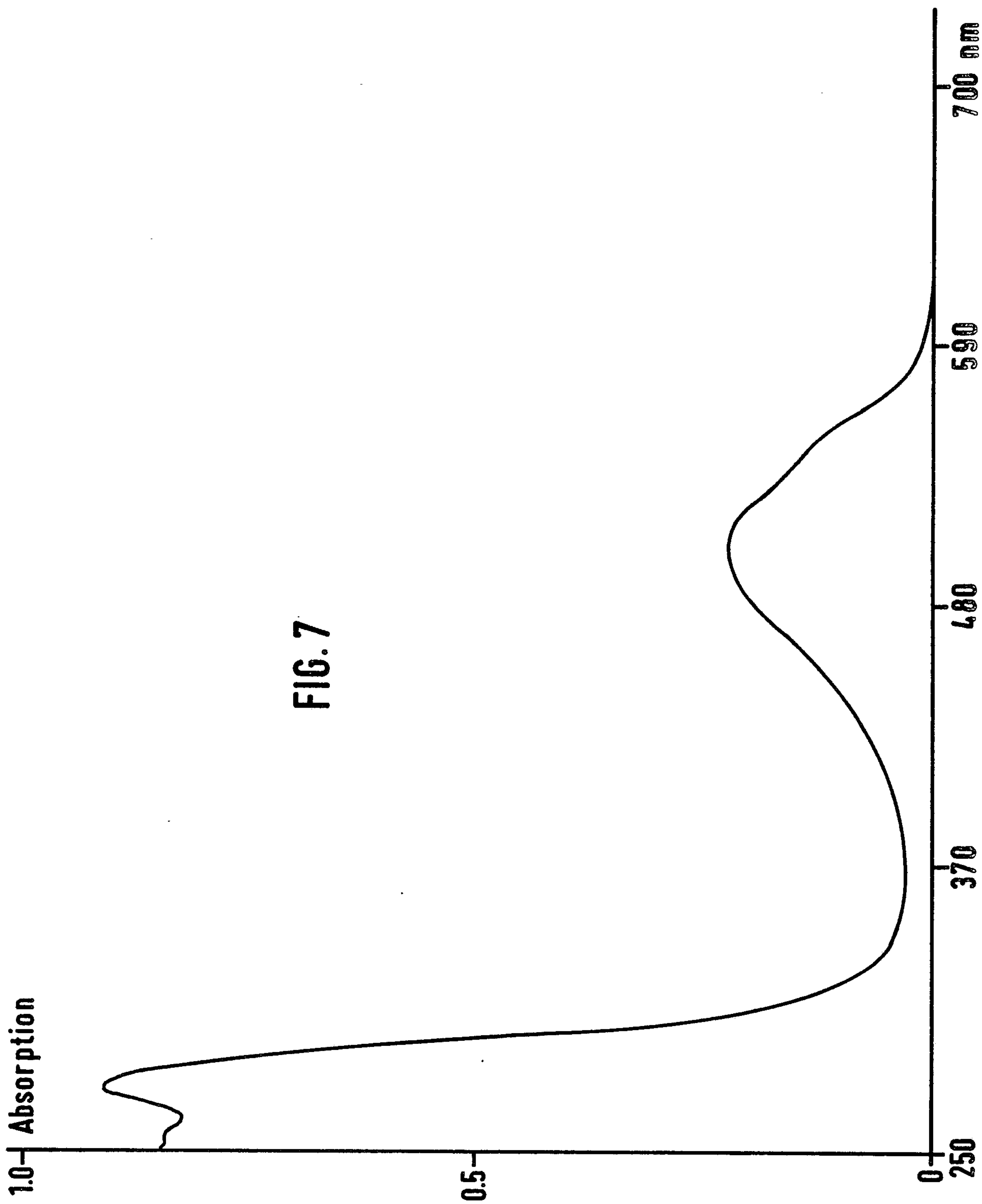


FIG. 7

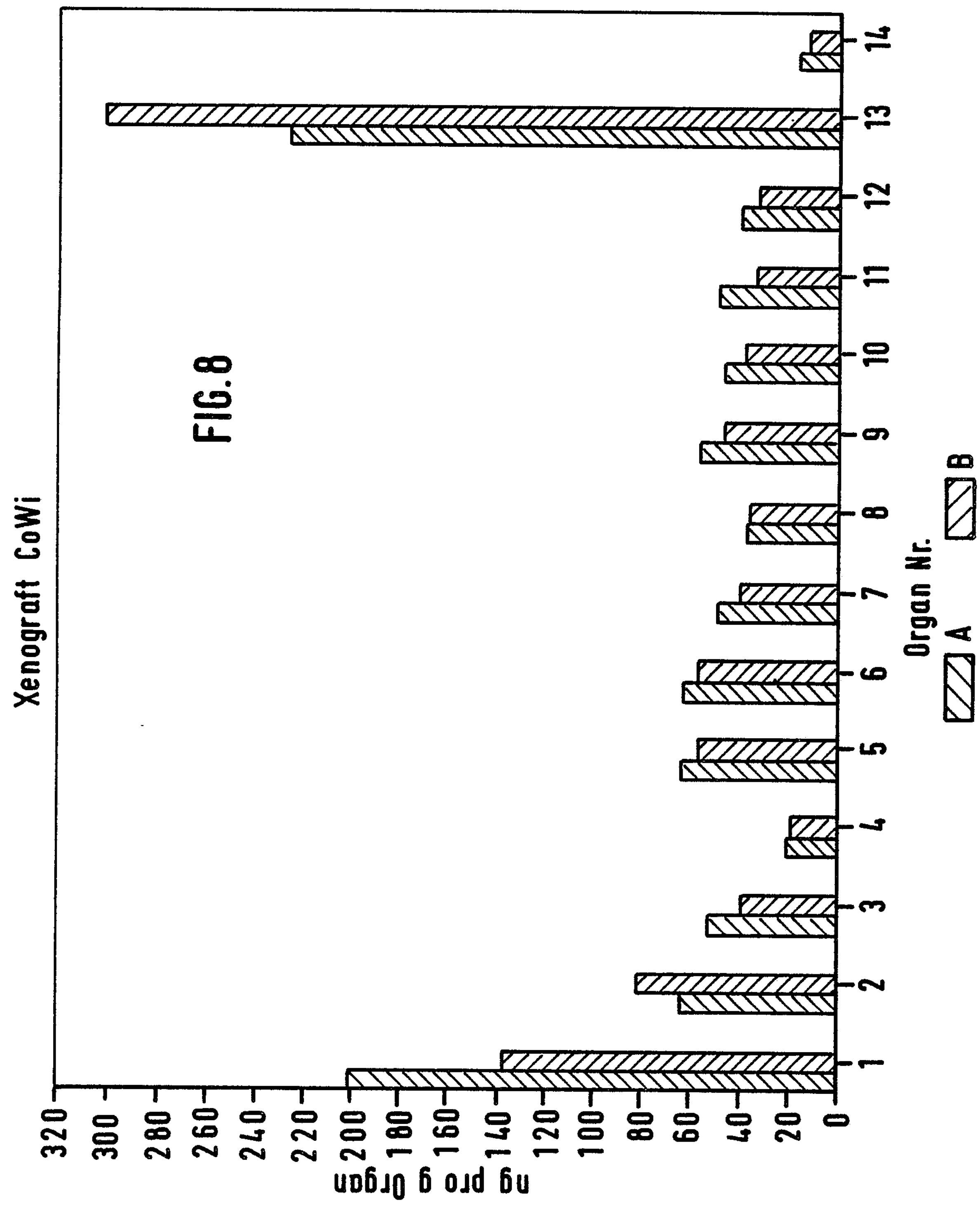
Xenograft CoWi
ng pro g Organ
0
20
40
60
80
100
120
140
160
180
200
220
240
260
280
300
320
1
2
3
4
5
6
7
8
9
10
11
12
13
14
Organ Nr.
A
B

FIG.8

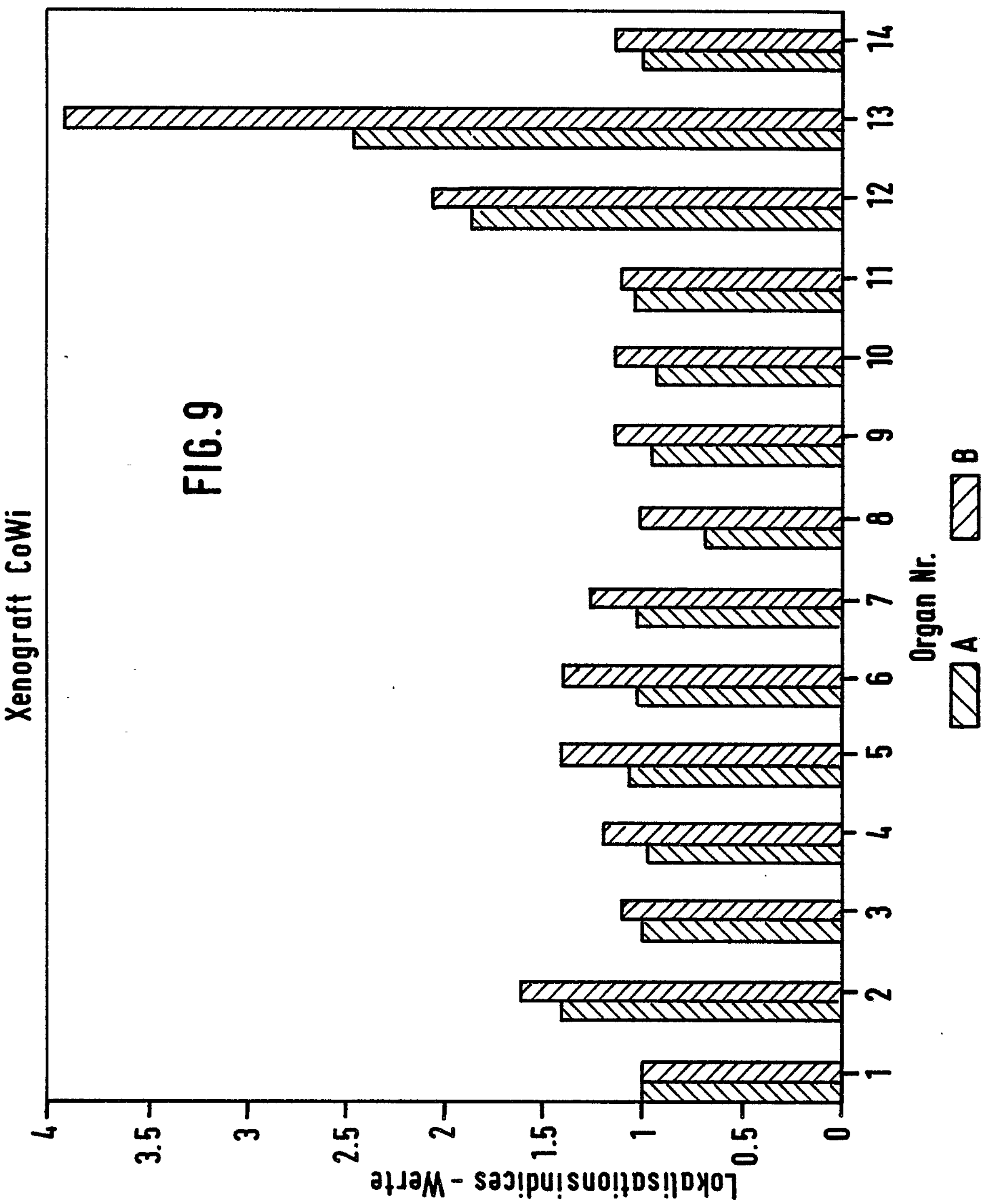
Xenograft CoWi
Lokalisationsindices - Werte
0
0.5
1
1.5
2
2.5
3
3.5
4
1
2
3
4
5
6
7
8
9
10
11
12
13
14
Organ Nr.
A
B

FIG. 9

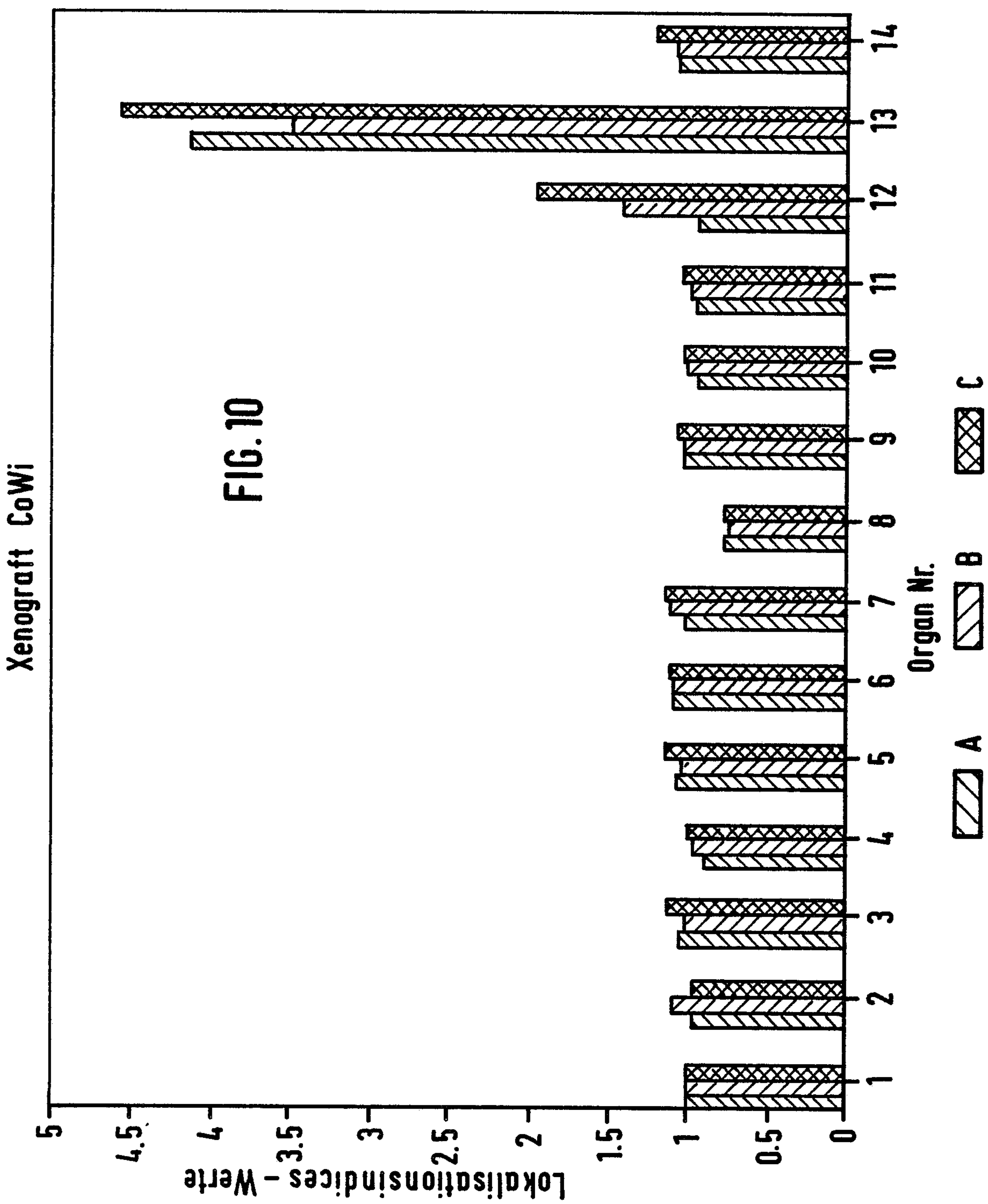
Xenograft CoWi
Lokalisationsindices - Werte
0
0.5
1
1.5
2
2.5
3
3.5
4
4.5
5
Organ Nr.
1
2
3
4
5
6
7
8
9
10
11
12
13
14
A
B
C

FIG. 10